# EUROPEAN PATENT APPLICATION

(11) **EP 4 254 206 A1**
(43) Date of publication of application: **04.10.2023**
(21) Application number: 20963424.5
(22) Date of filing: 24.11.2020
(51) Int. Cl.: G06F 13/00

(54) **DEVICE SERVING AS FLAVOR INHALER OR AS AEROSOL GENERATION DEVICE, MANAGEMENT COMPUTER, AND DATA CONVERSION COMPUTER**

(71) Applicant: Japan Tobacco Inc., Tokyo 105-6927 (JP)
(72) Inventor: YAMAMOTO, Kazushi, Tokyo 130-8603 (JP)
(74) Representative: Hoffmann Eitle
(86) International application number: PCT/JP2020/043592
(87) International publication number: WO 2022/113159

(57) **Abstract**

The present invention provides a flavor inhaler and the like whereby it is possible to adequately perform management (including control) using a communication standard with predetermined restrictions. This device serves as a flavor inhaler or as an aerosol generation device. The device is configured so that when control data for controlling the device is received While processing for transmitting data is on standby, the processing is deferred (910, 915), confirmation response data is transmitted (940), and the processing for transmitting data is executed (970).

## Description

### TECHNICAL FIELD

The present application relates to a flavor inhaler or an aerosol-generating device (hereinafter referred to as a "flavor inhaler or the like"). More particularly, the present application relates to a flavor inhaler or the like that communicates in accordance with an LPWA wireless communication standard or a wireless communication standard with similar limitations.

Note that a flavor inhaler refers to a tool for inhaling flavor, including but not limited to electronic tobacco products, heated tobacco products, and traditional tobacco products, for example. Also, an "aerosol-generating device" refers to a device for inhaling a generated aerosol, including but not limited to electronic tobacco products, heated tobacco products, and medical nebulizers, for example. Moreover, the flavor inhaler or the like includes what are called reduced-risk products (RRP).

### BACKGROUND ART

If a communication module conforming to an LPWA wireless communication standard is built into a flavor inhaler or the like, the flavor inhaler or the like can perform wireless communication independently without going through communication equipment such as a user's smartphone.

In this regard, PTL 1 discloses that when a fall or impact is detected by an accelerometer, an aerosol-generating device transmits data related to the fall or impact directly to a base station through an LPWA network.

PTL 2 discloses a method by which electronic tobacco products exchange information when communicating directly with each other.

PTL 3 and PTL 4 disclose methods by which electronic tobacco products exchange information about a flavor source with each other.

PTL 5 discloses that a consumable, aerosol-generating substrate is authenticated by RFID or a sensor in an aerosol-generating device.

### CITATION LIST

### PATENT LITERATURE

PTL 1: WO 2019/186468 A1
PTL 2: US 10,129,727 B2 (WO 2015/149340 A1 patent family)
PTL 3: US 2017/0118292 A1 (WO 2015/149339 A1 patent family)
PTL 4: US 10,123,565 B2 (WO 2015/149336 A1 patent family)
PTL 5: Japanese Translation of PCT International Application Publication No. 2018-522542 (WO 2016/199066 A1 patent family)

### SUMMARY OF INVENTION

### TECHNICAL PROBLEM

According to an LPWA wireless communication standard of the related art (for example, Sigfox), the data that can be transmitted and received by a device having a corresponding communication module built in is limited to data in a binary format (hereinafter referred to as "binary data"). Binary data transmitted from the device is transmitted to another computer after being converted into JSON data (data in a text format; hereinafter referred to as "text data") by a data conversion computer called a binary parser. The same is also true of communication from another computer to the device.

However, data conversion computers of the related art can only convert binary data in a single format. For a flavor inhaler or the like, there may be multiple formats of data expected to be transmitted and received, such as data in a format for initial communication and data in a format for normal communication. Consequently, there is a problem in that a data conversion computer of the related art is unable to deal with the conversion of such data that is expected in a flavor inhaler or the like.

Moreover, according to an LPWA wireless communication standard of the related art, there is no acknowledgment with respect to data transmission, and it is unknown whether transmitted data reaches a destination. This is because communication according to an LPWA wireless communication standard is assumed to be the transmission of simple data from sensors, such as gas or waterworks metering data, container tracking data and vinyl greenhouse temperature data, and there is not much demand for communication reliability.

Accordingly, in the case where communication is performed simply according to an LPWA wireless communication standard of the related art, there is a problem in that the communication is of lower reliability for use with a flavor inhaler or the like.

Furthermore, according to an LPWA wireless communication standard of the related art, there are limits on the size of the data that can be transmitted and received and the number of times that data can be transmitted and received per day by a device having a corresponding communication module built in. For example, according to Sigfox, the size of data that can be transmitted at one time is 12 bytes, the size of data that can be received is 8 bytes, the number of times that data can be transmitted per day is 140 times, and the number of times that data can be received is 4 times.

However, in a flavor inhaler or the like, it is anticipated that a variety of information will be transmitted and received, and thus in the case where communication is performed simply according to an LPWA wireless communication standard of the related art, there is a problem in that adequately controlling and managing the flavor inhaler or the like is difficult.

Incidentally, with regard to the method for externally ascertaining the type of refill (indicating the brand of tobacco, for example) for a cartridge, stick-type substrate, or the like (including a tobacco capsule) to be loaded as a replacement into a flavor inhaler or the like, in the related art, the method involves the flavor inhaler or the like itself determining the type of refill on the basis of output from a sensor or the like, and transmitting relevant information to an external computer. However, according to an LPWA wireless communication standard of the related art, there is a problem regarding how the type of refill is to be transmitted under the limitations on data transmission described above. Additionally, in the above method, it is necessary for the flavor inhaler or the like to store correspondence relationships between the sensor output and the type of refill, but according to an LPWA wireless communication standard of the related art, there is a problem in that, when a new type of refill is introduced or the like, updating the correspondence relationships is difficult under the limitations on data reception described above.

The present invention has been devised in light of the above, and the objective thereof is to provide a flavor inhaler or the like, a data conversion computer, a management computer, a method, a program, and the like that address the problems described above.

### SOLUTION TO PROBLEM

The present invention has been devised in light of the above.

To address at least one of the problems described above, according to a first aspect of the present invention, there is provided a data conversion computer for generating data in a second format on a basis of data in a first format, the data conversion computer being configured to: obtain data being a predetermined first portion of the data in the first format; and generate the data in the second format on a basis of data being one or more second portions of the data in the first format; wherein which portion of the data in the first format is each of the second portions is determined on a basis of the obtained data being the first portion.

In the data conversion computer according to the first aspect, the generating of the data in the second format may comprise executing a different process for generating the data in the second format for each type of the data being the first portion.

In the data conversion computer according to the first aspect, the generating of the data in the second format may include determining a position and size of each second portion in the data in the first format on a basis of the first portion of data.

The data conversion computer according to the first aspect may be further configured such that the position and size of each of the second portions in the data in the first format are determined in units of bytes or in units of bits.

In the data conversion computer according to the first aspect, the generating of the data in the second format may comprise generating a string on a basis of a name corresponding to each of the second portions and the data being each of the second portions.

In the data conversion computer according to the first aspect, the generating of the data in the second format may comprise obtaining a part of the data in the second format by converting the data being a predetermined one of the second portions into a numeral expressed in decimal.

In the data conversion computer according to the first aspect, the data being each of the second portions may include a name, and the generating of the data in the second format may comprise determining a position and size, which correspond to each of the second portions, in the data in the second format on a basis of the data being the first portion and the name included in the data being each of the second portions.

The data conversion computer according to the first aspect may be further configured such that the position and size, which correspond to each of the second portions, in the data in the second format are determined in units of bytes or in units of bits.

In the data conversion computer according to the first aspect, data being each of the second portions may include a value, and the generating of the data in the second format may comprise obtaining a part of the data in the second format by converting the data being a predetermined one of the second portions into a numerical value.

In the data conversion computer according to the first aspect, data being each of the second portions may include a value, and the generating of the data in the second format may comprise padding data such that the data in the second format is a predetermined size.

To address at least one of the problems described above, according to a second aspect of the present invention, there is provided a system comprising a device which is a flavor inhaler or an aerosol-generating device, the data conversion computer according to the first aspect, and a management computer, the system being configured such that: the device transmits data directly or indirectly to the data conversion computer and/or receives data directly or indirectly from the data conversion computer in accordance with a predetermined LPWA wireless communication standard, the data being one of the data in a first format and the data in the second format; and the management computer transmits data directly or indirectly to the data conversion computer and/or receives data directly or indirectly from the data conversion computer, the data being the other of the data in the first format and the data in the second format.

To address at least one of the problems described above, according to a third aspect of the present invention, there is provided a method, executed by a computer, for generating data in a second format on a basis of data in a first format, the method comprising: obtaining data being a predetermined first portion of the data in the first format; and generating the data in the second format on a basis of data being one or more second portions of the data in the first format, wherein which portion of the data in the first format is each of the second portions is determined on a basis of the obtained data of the first portion.

To address at least one of the problems described above, according to a fourth aspect of the present invention, there is provided a program causing a computer to execute the method according to the third aspect.

To address at least one of the problems described above, according to a fifth aspect of the present invention, there is provided a device which is a flavor inhaler or an aerosol-generating device, the device being configured to: generate data including a session number; execute a process for transmitting the generated data, and also store the data; and execute, if a retransmission request specifying the session number is received, a process for retransmitting stored data including the specified session number.

In the device according to the fifth aspect, the generating of data comprising the session number may include generating session numbers such that the generated session numbers are cyclical, and the device according to the fifth aspect may be further configured such that a plurality of data including the same session number is not stored.

In the device according to the fifth aspect, the executing of the process for retransmitting stored data including the specified session number may comprise: deferring, if a process for transmitting different data is on standby, the execution of the process; and executing the process for transmitting the different data if the retransmission of stored data including the specified session number yields a predetermined result.

The device according to the fifth aspect may be further configured to: generate data that does not include the session number, and execute a process for transmitting the generated data, and wherein the data that does not include the session number is not stored.

The device according to the fifth aspect may be further configured to generate data indicating a type of refill mounted in the device and/or data indicating an error that has occurred in the device as the data including the session number.

To address at least one of the problems described above, according to a sixth aspect of the present invention, there is provided a management computer that communicates with a device which is a flavor inhaler or an aerosol-generating device, the management computer being configured to: when data including a session number is received, determine on a basis of the session number whether data that should be received before the data including the session number has been received; if it is determined that the data that should be received before the data including the session number has not been received, determine one or more session numbers included respectively in one or more sets of the data that should be received but has not been received; and issue a retransmission request specifying the determined one or more session numbers.

The management computer according to the sixth aspect may be configured such that the session number is generated consecutively and cyclically, and a single retransmission request specifies a minimum value and a maximum value of the determined one or more session numbers.

To address at least one of the problems described above, according to a seventh aspect of the present invention, there is provided a system comprising: the device according to the fifth aspect; and the management computer according to the sixth aspect, wherein the device is configured to communicate indirectly with the management computer in accordance with a predetermined LPWA wireless communication standard.

To address at least one of the problems described above, according to an eighth aspect of the present invention, there is provided a method executed by a device which is a flavor inhaler or an aerosol-generating device, the method comprising: generating data including a session number; executing a process for transmitting the generated data, and also storing the data; and executing, if a retransmission request specifying a session number is received, a process for retransmitting stored data including the specified session number.

To address at least one of the problems described above, according to a ninth aspect of the present invention, there is provided a method executed by a computer that communicates with a device which is a flavor inhaler or an aerosol-generating device, the method comprising: when data including a session number is received, determining on a basis of the session number whether data that should be received before the data including the session number has been received; if it is determined that the data that should be received before the data including the session number has not been received, determining one or more session numbers included respectively in one or more sets of the data that should be received but has not been received; and issuing a retransmission request specifying the determined one or more session numbers.

To address at least one of the problems described above, according to a 10th aspect of the present invention, there is provided a program causing a device which is a flavor inhaler or an aerosol-generating device to execute the method according to the eighth aspect.

To address at least one of the problems described above, according to an 11th aspect of the present invention, there is provided a program causing a computer to execute the method according to the ninth aspect.

To address at least one of the problems described above, according to a 12th aspect of the present invention, there is provided a device which is a flavor inhaler or an aerosol-generating device, the device being configured to: defer a process for transmitting data, if control data for controlling the device is received when the process is on standby; transmit acknowledgment data; and execute the process for transmitting data.

The device according to the 12th aspect may be further configured to: obtain data which is at a predetermined position in the control data and which has a predetermined size; perform a first control on a basis of first data from the control data, wherein the size of the first data is one or more bytes, the first data is in units of bytes, and the size and a position of the first data in the control data is determined on a basis of the obtained data; and perform a second control on a basis of second data, which is one bit, from the control data, wherein a position of the of second data in the control data is determined on a basis of the obtained data.

In the device according to the 12th aspect, the first data may include a message encoded in extended ASCII code, and the first control may include a message display.

The device according to the 12th aspect may be further configured to perform communication in accordance with a predetermined LPWA wireless communication standard at least to receive the control data and the transmit acknowledgment data.

To address at least one of the problems described above, according to a 13th aspect of the present invention, there is provided a management computer configured to: transmit control data for controlling a device which is a flavor inhaler or an aerosol-generating device, wherein the transmitted control data is retransmitted if acknowledgment data is not received within a predetermined time from the transmission of the control data.

The management computer according to the 13th aspect may be further configured to: transmit control data including a portion of an encoded message; and if the acknowledgment data is received within a predetermined time from the transmission of the control data, transmit control data including a different portion of the encoded message.

To address at least one of the problems described above, according to a 14th aspect of the present invention, there is provided a data conversion computer for generating control data in a first format on a basis of control data in a second format to control a device which is a flavor inhaler or an aerosol-generating device, the data conversion computer being configured to: generate, on a basis of data being a first portion of the control data in the second format, first data the size of which is one or more bytes and which is in units of bytes; generate, on a basis of data being a second portion of the control data in the second format, second data, the size of which is one bit; generate, on a basis of one or more sets of second data, third data, the size of which is one byte; and generate, on a basis of the first data and the third data, the control data in the first format.

The data conversion computer according to the 14th aspect may be further configured to; obtain data being a predetermined third portion of the control data in the second format, wherein which portions of the control data in the second format are the first portion and the second portion are determined on a basis of the obtained data being the third portion, the generating of third data, the size of which is one byte, includes: arranging the one or more sets of second data in an order determined on a basis of the obtained data being the third portion, and the control data in the first format is generated also on a basis of the obtained data being the third portion.

To address at least one of the problems described above, according to a 15th aspect of the present invention, there is provided a system comprising: the device according to the 12th aspect; the management computer according to the 13th aspect; and the data conversion computer according to the 14th aspect, wherein control data received by the device is the control data in the first format generated by the data conversion computer, and control data in the second format used by the data conversion computer is the control data transmitted by the management computer.

To address at least one of the problems described above, according to a 16th aspect of the present invention, there is provided a method executed by a device which is a flavor inhaler or an aerosol-generating device, the method comprising: deferring a process for transmitting data, if control data for controlling the device is received when the process is on standby; transmitting acknowledgment data; and executing the process for transmitting data.

To address at least one of the problems described above, according to a 17th aspect of the present invention, there is provided a method executed by a computer, the method comprising: transmitting control data for controlling a device which is a flavor inhaler or an aerosol-generating device; and retransmitting the transmitted control data if acknowledgment data is not received within a predetermined time from the transmission of the control data.

To address at least one of the problems described above, according to an 18th aspect of the present invention, there is provided a method executed by a computer for generating control data in a first format on a basis of control data in a second format to control a device which is a flavor inhaler or an aerosol-generating device, the method comprising: generating, on a basis of data being a first portion of the control data in the second format, first data, the size of which is one or more bytes and which is in units of bytes; generating, on a basis of data being a second portion of the control data in the second format, second data, the size of which is one bit; generating, on a basis of one or more sets of the second data, third data, the size of which is one byte; and generating, on a basis of the first data and the third data, the control data in the first format.

To address at least one of the problems described above, according to a 19th aspect of the present invention, there is provided a program causing a device which is a flavor inhaler or an aerosol-generating device to execute the method according to the 16th aspect.

To address at least one of the problems described above, according to a 20th aspect of the present invention, there is provided a program causing a computer to execute the method according to the 17th or 18th aspect.

To address at least one of the problems described above, according to a 21st aspect of the present invention, there is provided a device which is a flavor inhaler or an aerosol-generating device, the device comprising: a color sensor configured to sense a color of at least a part of a component removably mounted in the device; and a communication unit configured to transmit data indicating the color sensed by the color sensor.

The device according to the 21st aspect may be configured such that the data includes a plurality of values specifying the color sensed by the color sensor.

The device according to the 21st aspect may be configured such that the color sensor includes one of the following: a white LED and a sensor capable of sensing the intensity of each of three primary colors RGB; or an LED capable of emitting light in each of three primary colors RGB individually and a photosensor.

In the device according to the 21st aspect, the component may be a refill.

To address at least one of the problems described above, according to a 22nd aspect of the present invention, there is provided a management computer configured to: receive data indicating a color of at least a part of a component removably mounted in a device which is a flavor inhaler or an aerosol-generating device; and determine a type of the component on a basis of the data.

In the management computer according to the 22nd aspect, the determining of a type of the component on a basis of the data may include determining a type of the component on a basis of the data and a data structure in which color corresponding to each type of component is indicated.

In the management computer according to the 22nd aspect, the color corresponding to a single type of component may include one standard color and one or more approximate colors.

In the management computer according to the 22nd aspect, the data may include a plurality of values specifying the color sensed by the color sensor, and the data structure may include, for each type of component, a plurality of values specifying the one corresponding standard color and a plurality of values specifying each corresponding approximate color.

In the management computer according to the 22nd aspect, the component may be a refill.

To address at least one of the problems described above, according to a 23rd aspect of the present invention, there is provided a system comprising: the device according to the 21st aspect; and the management computer according to the 22nd aspect.

In the system according to the 23rd aspect, the device may be configured to communicate with the management computer in accordance with a predetermined LPWA wireless communication standard.

To address at least one of the problems described above, according to a 24th aspect of the present invention, there is provided a method executed by a computer, the method comprising: receiving data indicating a color of at least a part of a component removably mounted in a device which is a flavor inhaler or an aerosol-generating device; and determining a type of the component on a basis of the data.

To address at least one of the problems described above, according to a 24th aspect of the present invention, there is provided a program causing a computer to execute the method according to the 23rd aspect.

### ADVANTAGEOUS EFFECTS OF INVENTION

According to an aspect of the present invention, another computer can manage (including control) a flavor inhaler or the like using an LPWA wireless communication standard of the related art or a wireless communication standard with similar limitations.

According to an aspect of the present invention, sufficiently reliable information transmission with respect to a flavor inhaler or the like can be achieved using an LPWA wireless communication standard of the related art or a wireless communication standard with similar limitations.

According to an aspect of the present invention, a flavor inhaler or the like can be managed sufficiently using an LPWA wireless communication standard of the related art or a wireless communication standard with similar limitations.

According to an aspect of the present invention, an external computer can manage the type of refill mounted in a flavor inhaler or the like using an LPWA wireless communication standard of the related art or a wireless communication standard with similar limitations.

### BRIEF DESCRIPTION OF DRAWINGS

Fig. 1A is a diagram schematically illustrating a first configuration example of a flavor inhaler or the like.
Fig. 1B is a diagram schematically illustrating a second configuration example of a flavor inhaler or the like.
Fig. 2 is a diagram schematically illustrating a configuration example of an overall system including a flavor inhaler or the like.
Fig. 3A illustrates a data format for initial communication.
Fig. 3B illustrates a data format for normal communication.
Fig. 3C illustrates a data format for emergency communication.
Fig. 3D illustrates a data format for acknowledgment of various settings request.
Fig. 3E illustrates a data format for acknowledgment of a message display request.
Fig. 3F illustrates a data format for request of time setting.
Fig. 3G illustrates a data format for request of various settings.

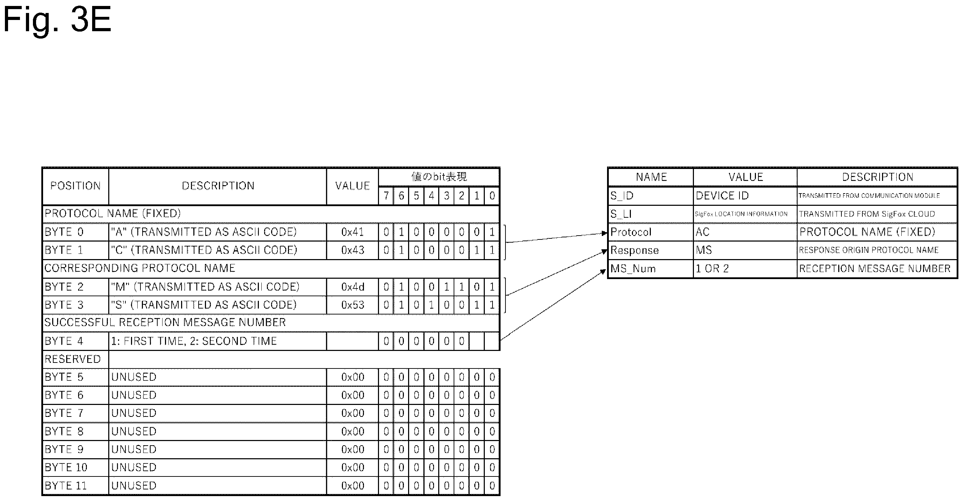

Fig. 3E illustrates a data format for request of a message display.

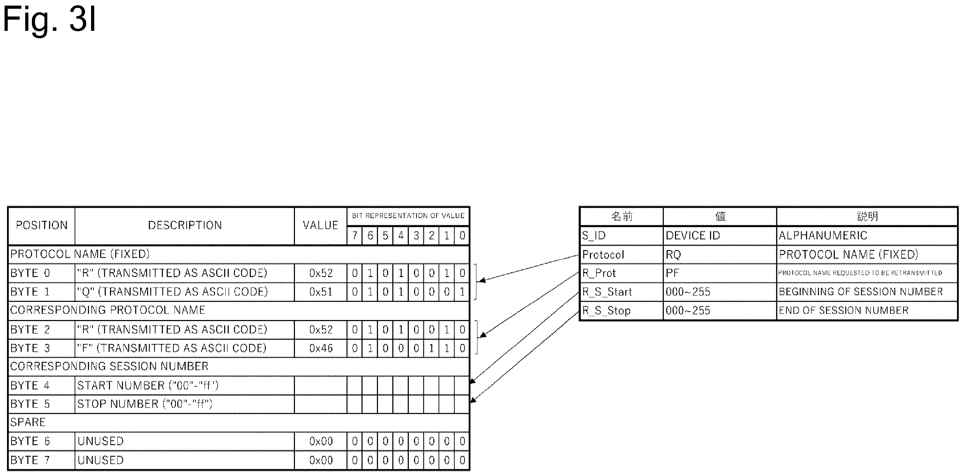

Fig. 3I illustrates a data format for requesting retransmission of refill data.

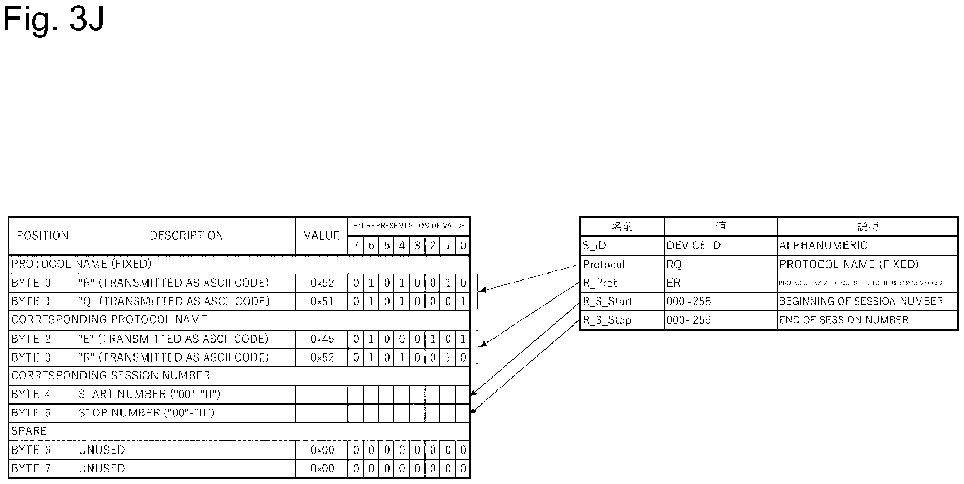

Fig. 3J illustrates a data format for requesting retransmission of error data.

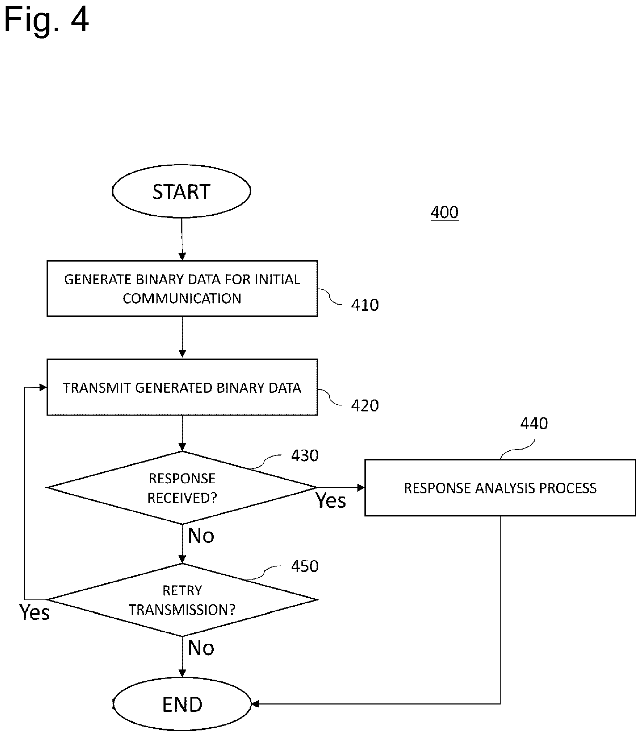

Fig. 4 is a flowchart of an initial communication process.

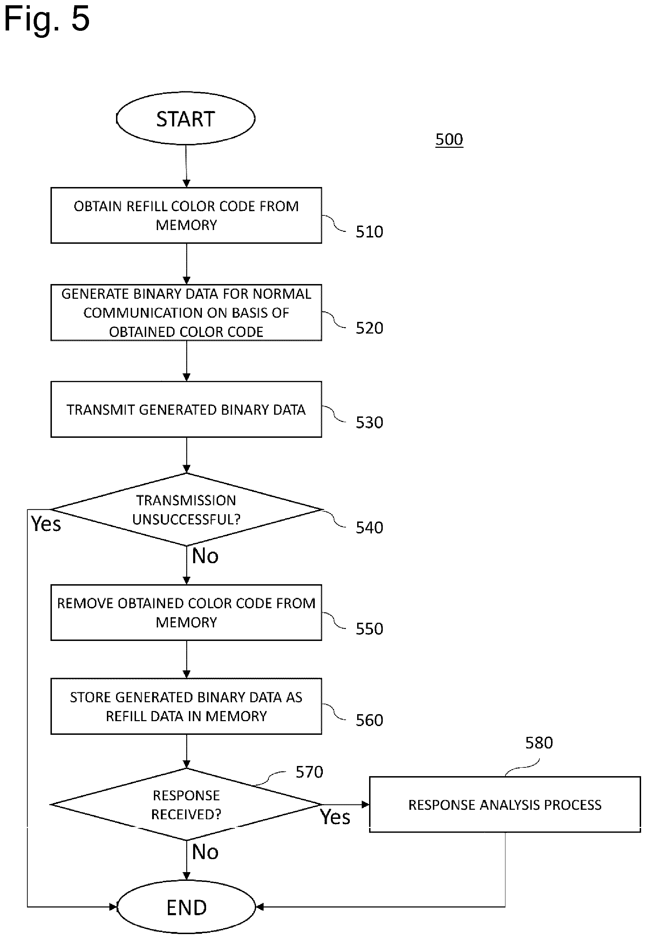

Fig. 5 is a flowchart of a normal communication process.

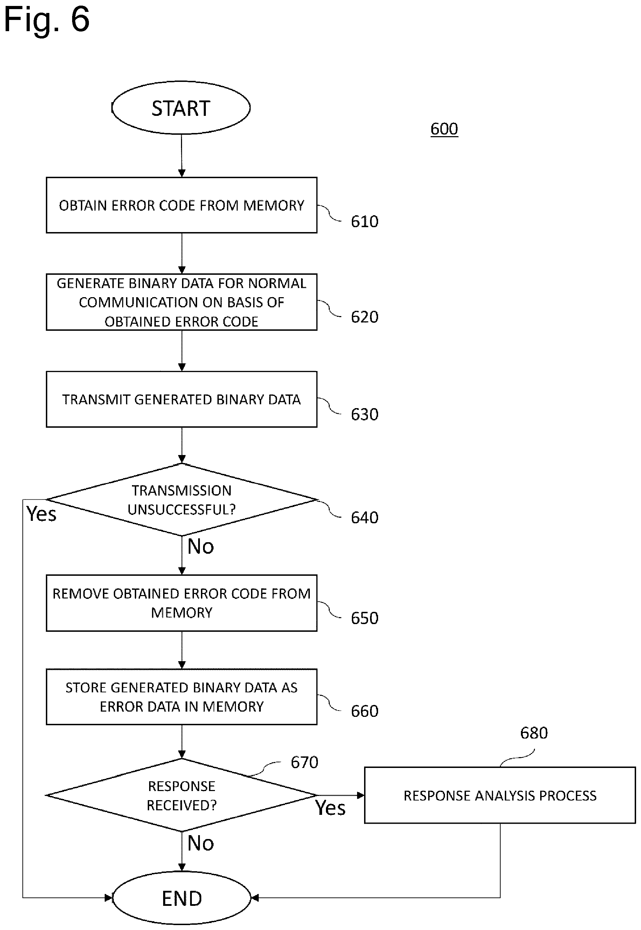

Fig. 6 is a flowchart of an emergency initial communication process.

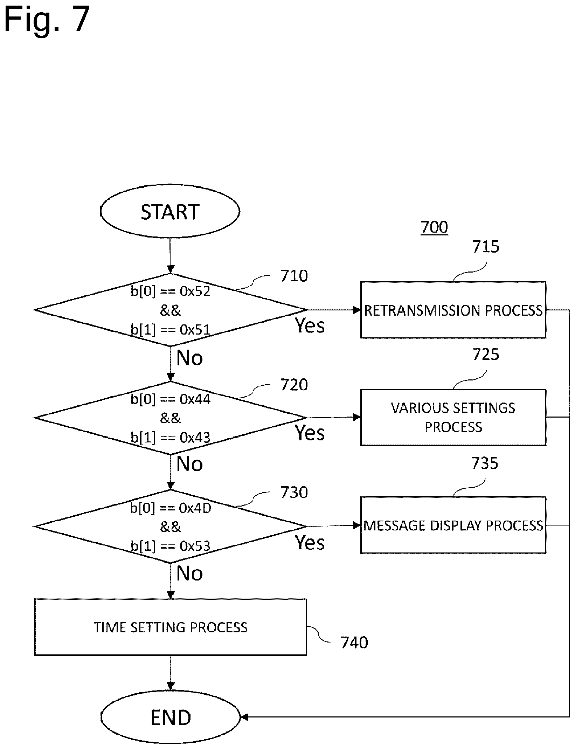

Fig. 7 is a flowchart of a response analysis process.

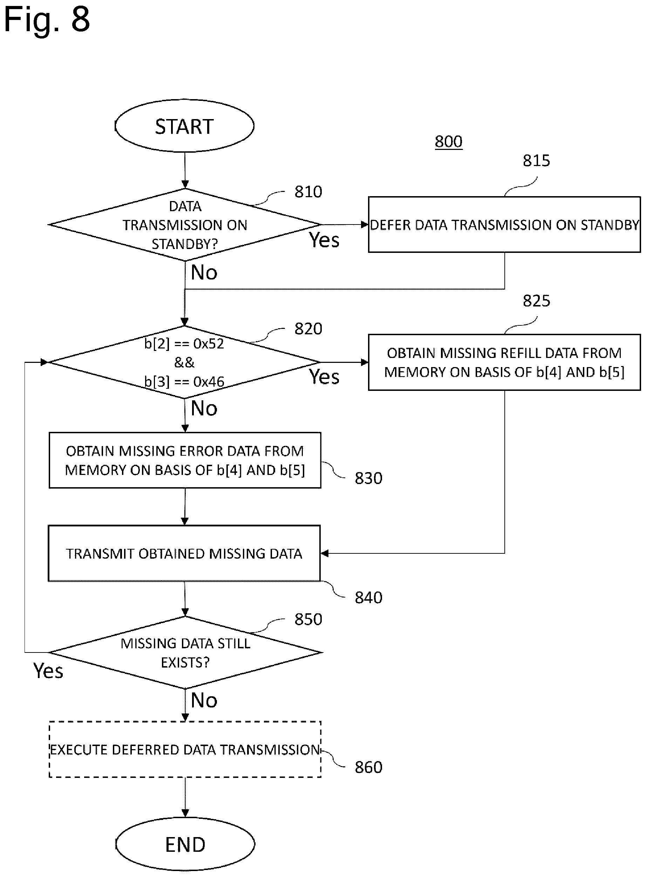

Fig. 8 is a flowchart of a retransmission process.

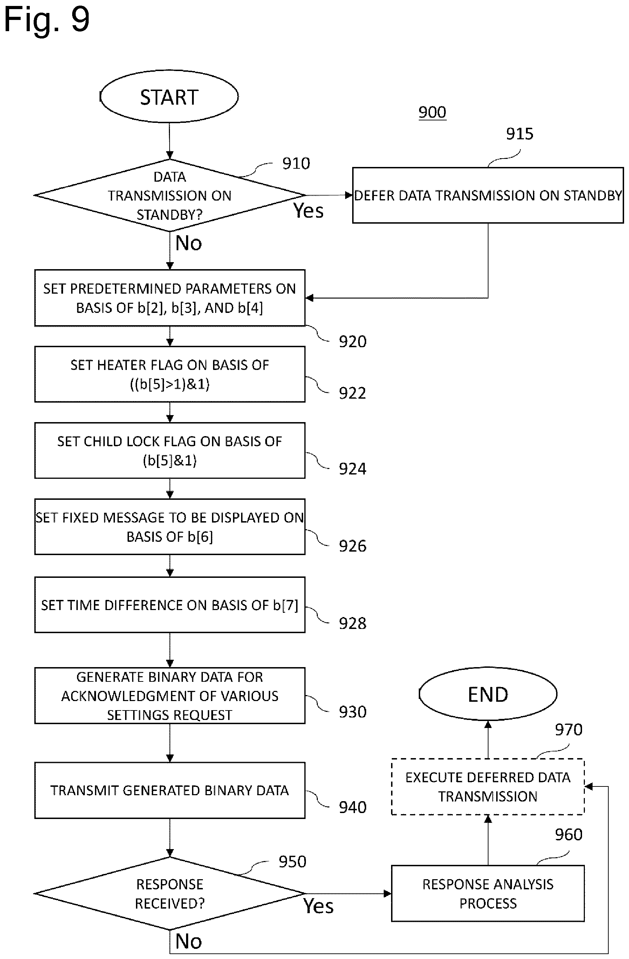

Fig. 9 is a flowchart of a various settings process.

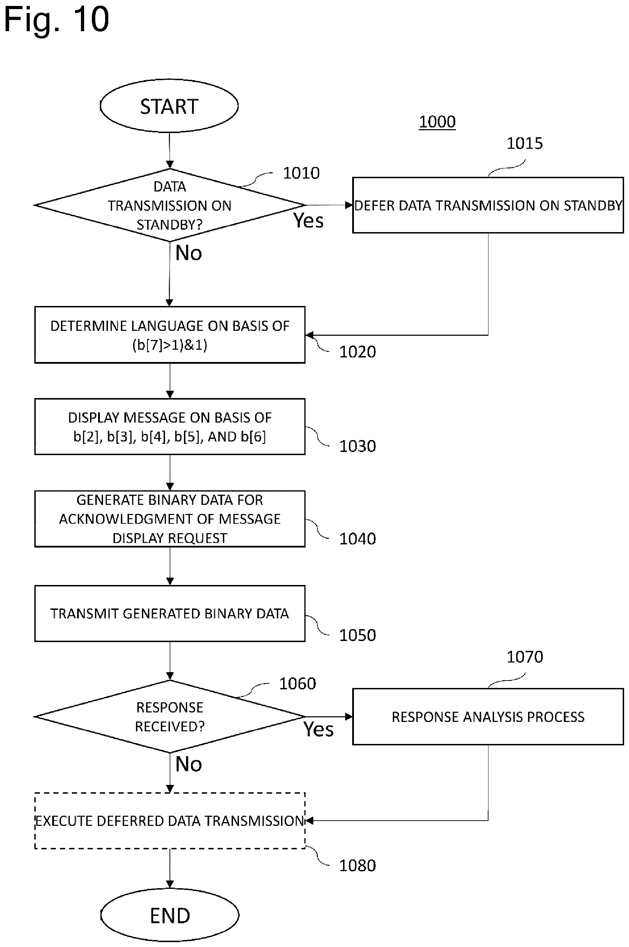

Fig. 10 is a flowchart of a message display process.

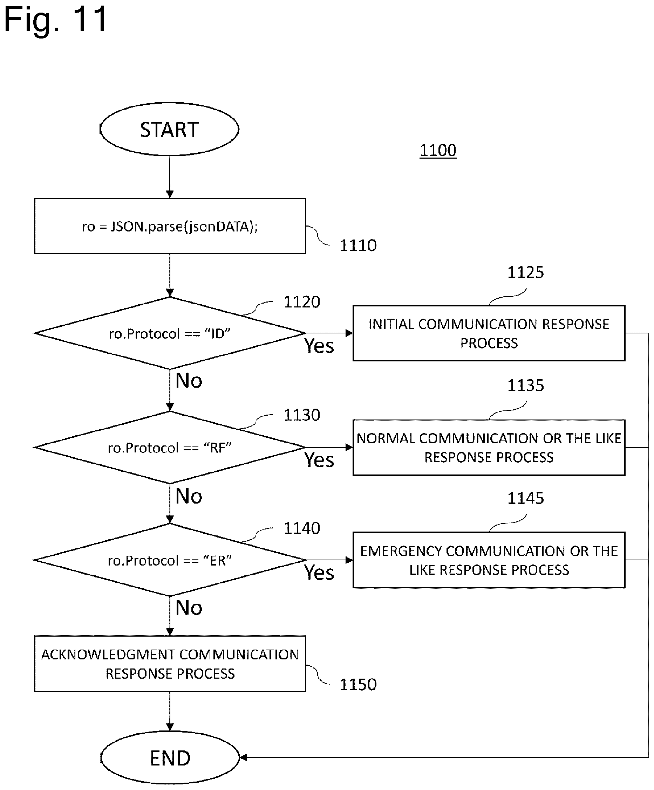

Fig. 11 is a flowchart of a response process.

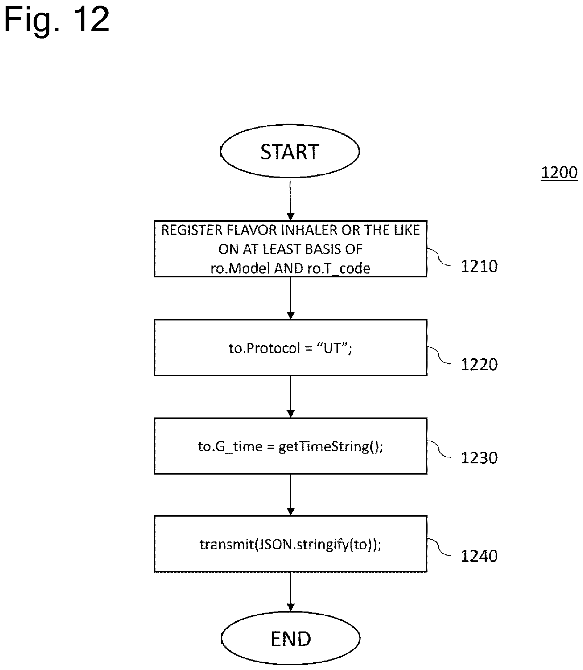

Fig. 12 is a flowchart of an initial communication response process.

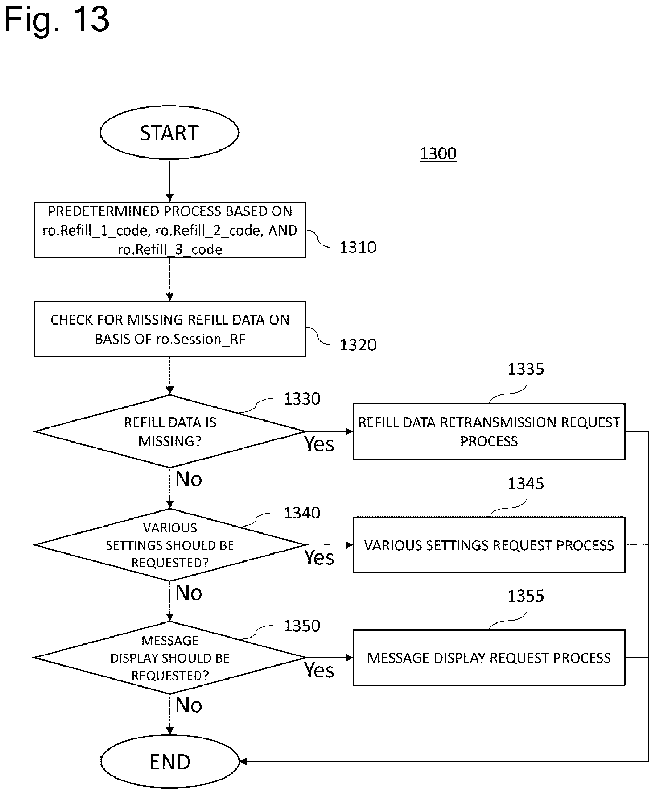

Fig. 13 is a flowchart of a normal communication or the like response process.

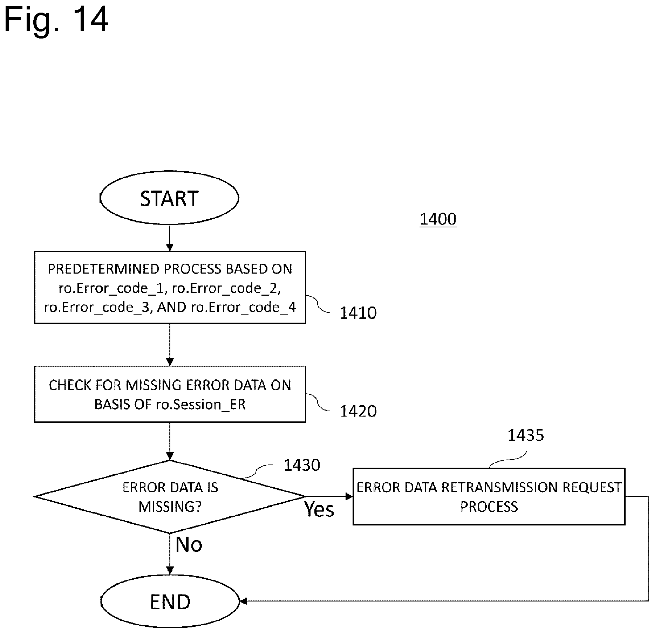

Fig. 14 is a flowchart of an emergency communication or the like response process.

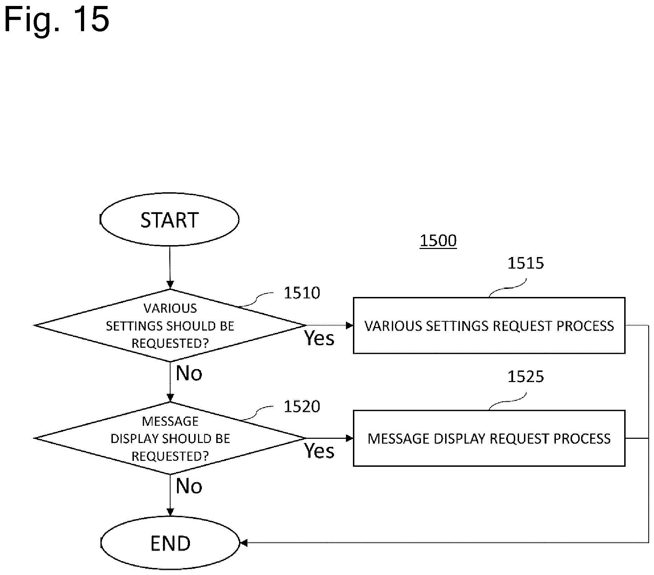

Fig. 15 is a flowchart of an acknowledgment communication response process.

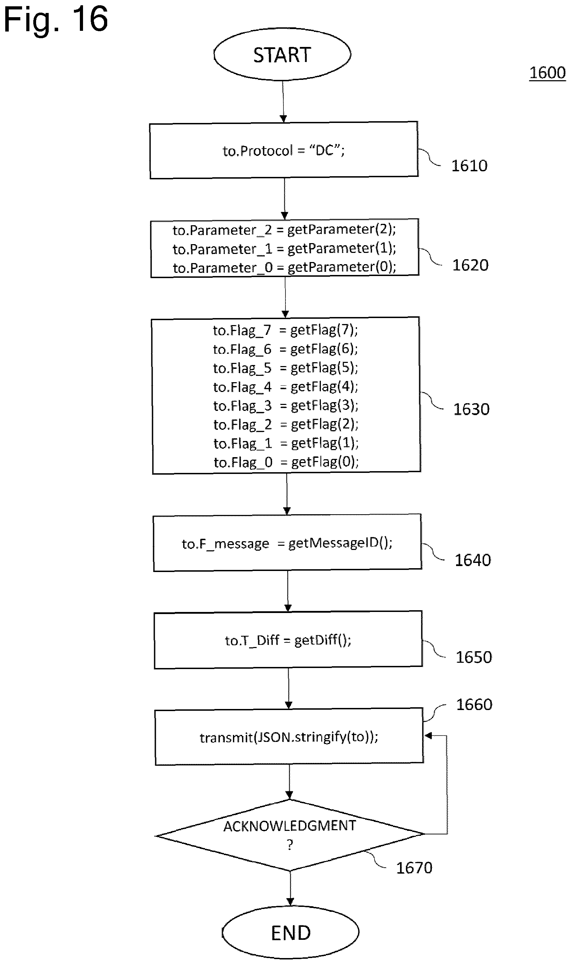

Fig. 16 is a flowchart of a various settings request process initial communication process.

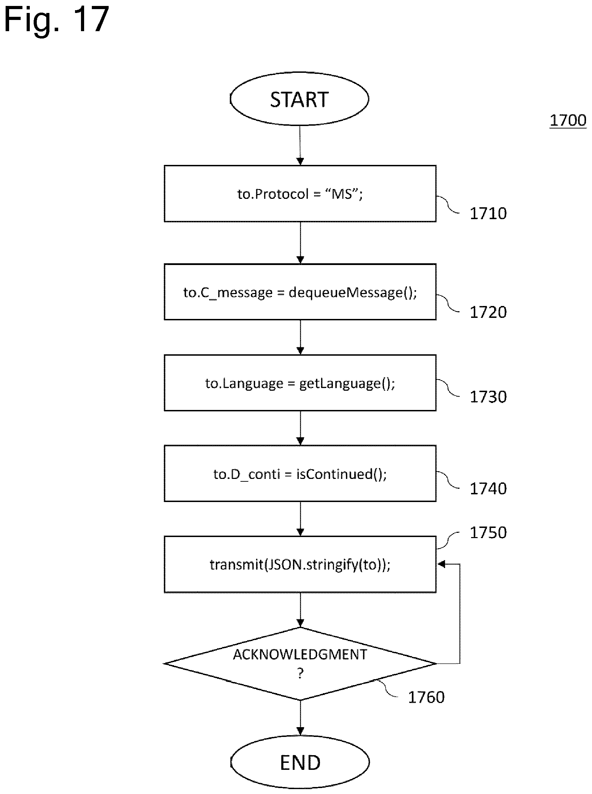

Fig. 17 is a flowchart of a message display request process.

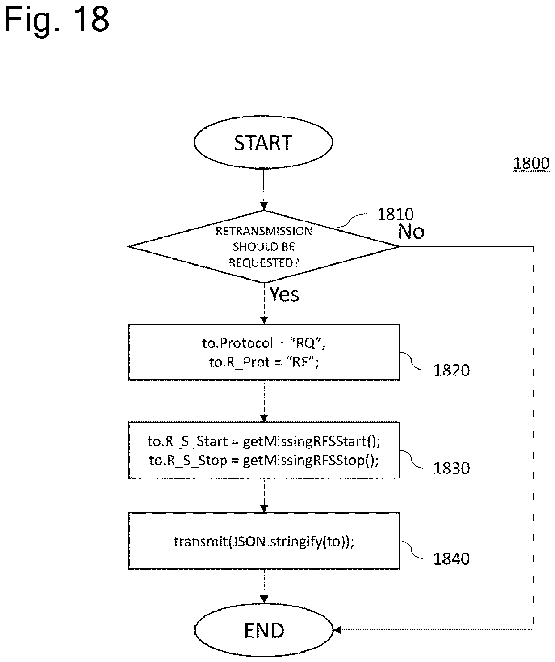

Fig. 18 is a flowchart of a refill data retransmission request process.

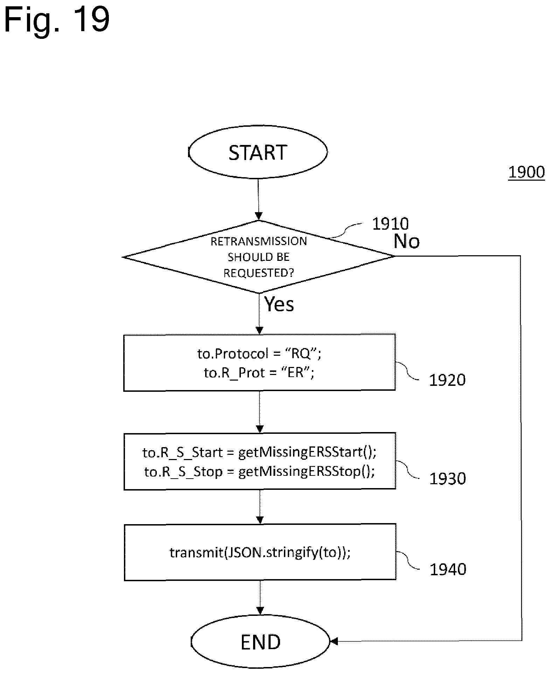

Fig. 19 is a flowchart of an error data retransmission request process.

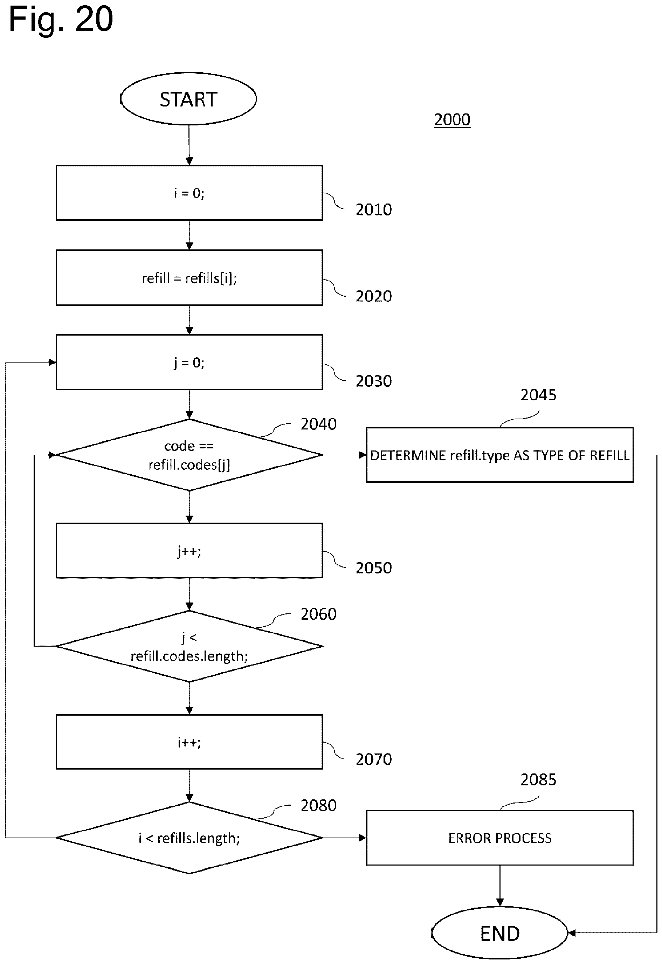

Fig. 20 is a flowchart of a refill type identification process.

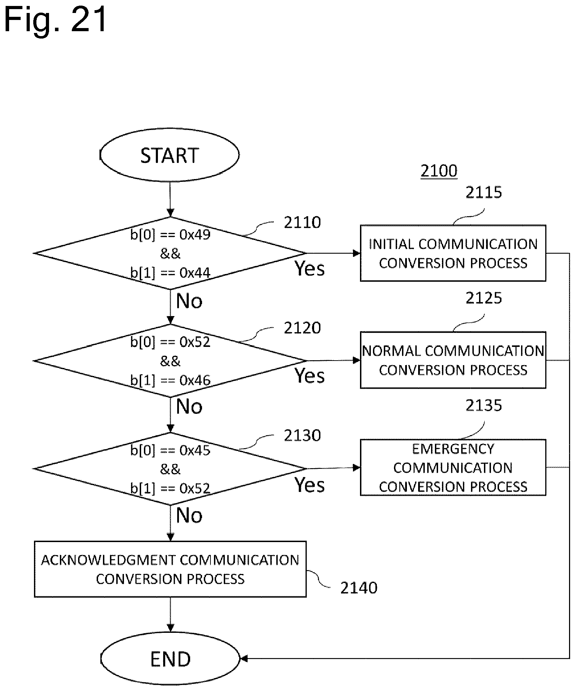

Fig. 21 is a flowchart of an upstream conversion process.

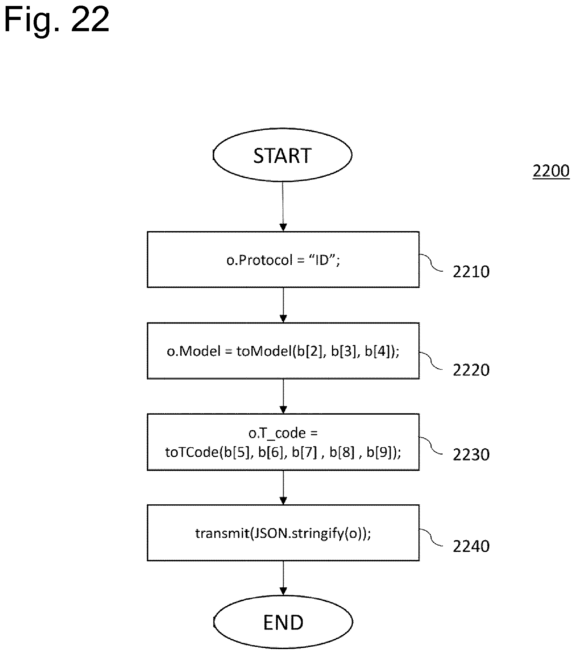

Fig. 22 is a flowchart of an initial communication conversion process.

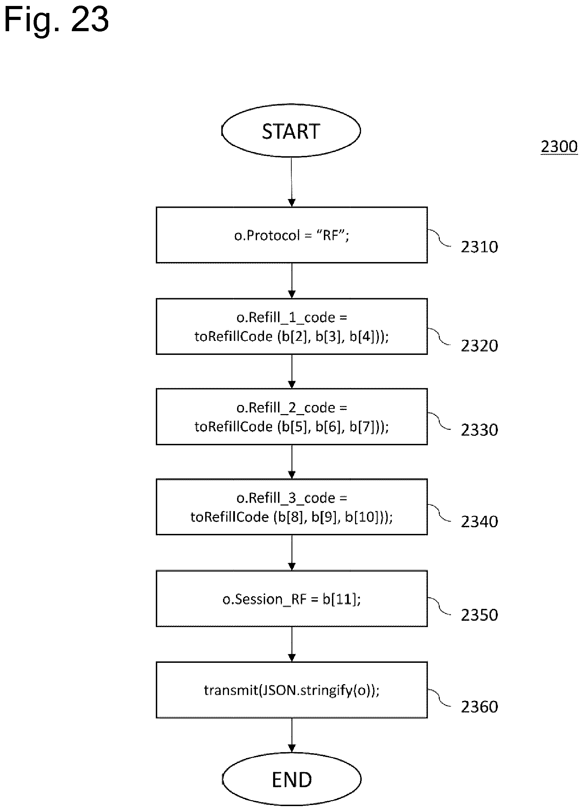

Fig. 23 is a flowchart of a normal communication conversion process.

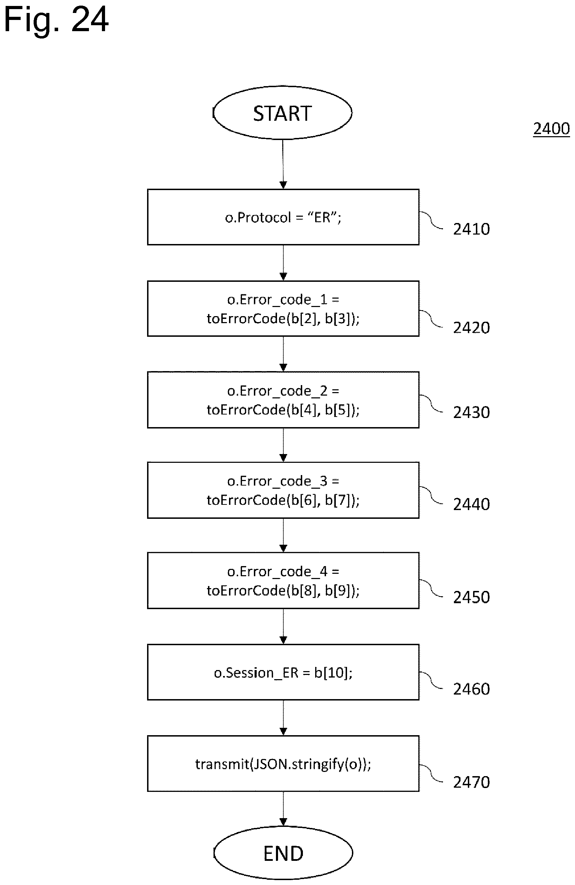

Fig. 24 is a flowchart of an emergency communication conversion process.

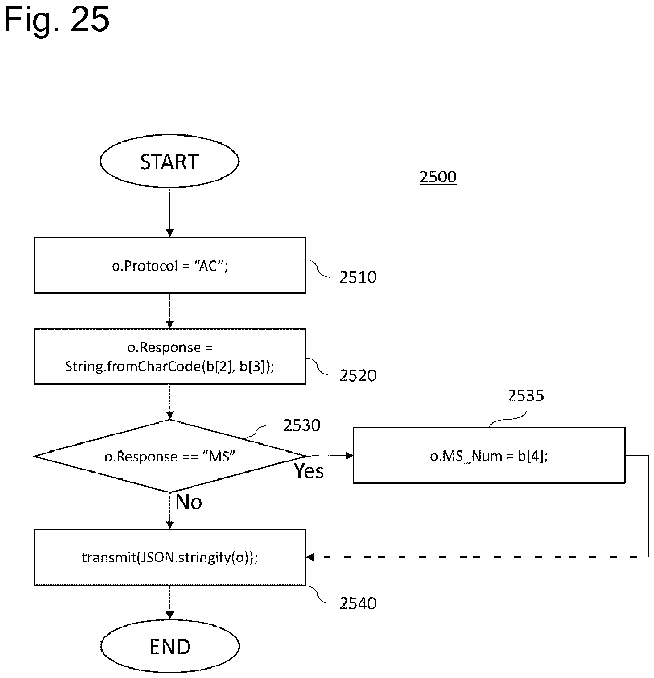

Fig. 25 is a flowchart of an acknowledgment communication conversion process.

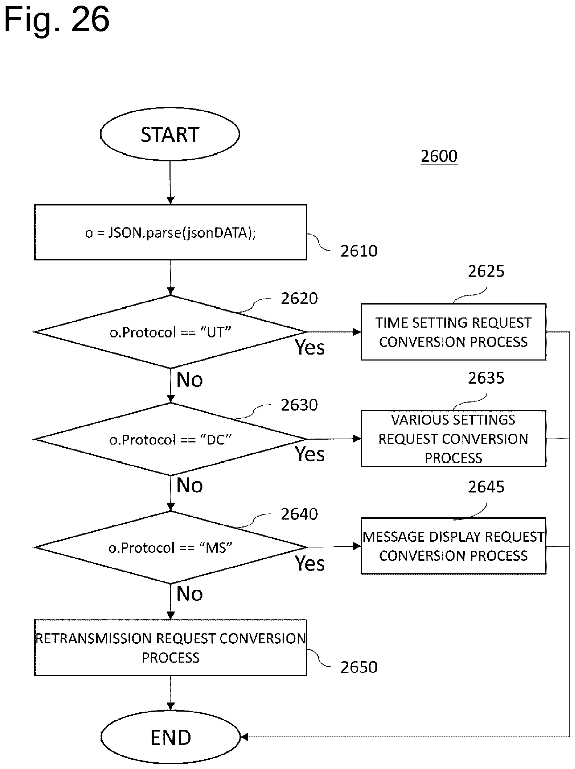

Fig. 26 is a flowchart of a downstream conversion process.

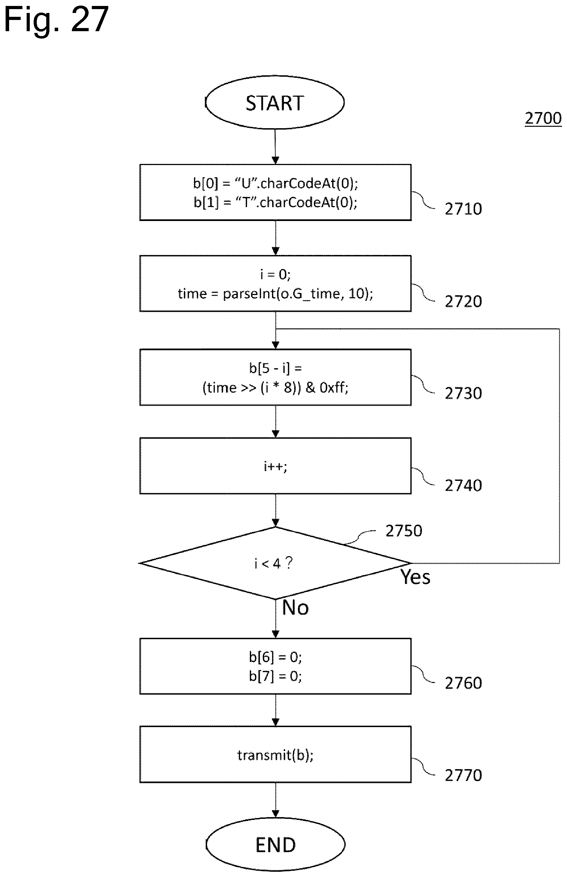

Fig. 27 is a flowchart of a time setting request conversion process.

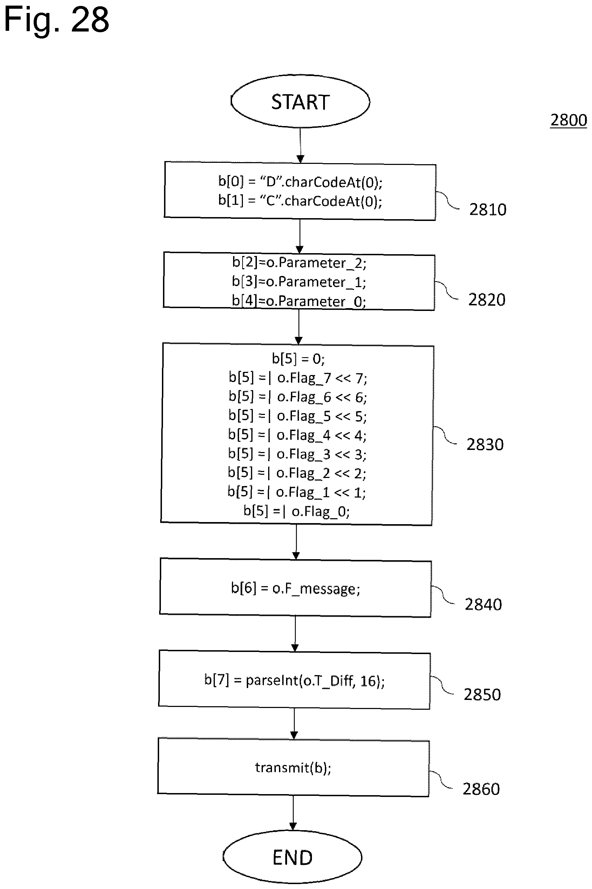

Fig. 28 is a flowchart of a various settings request conversion process.

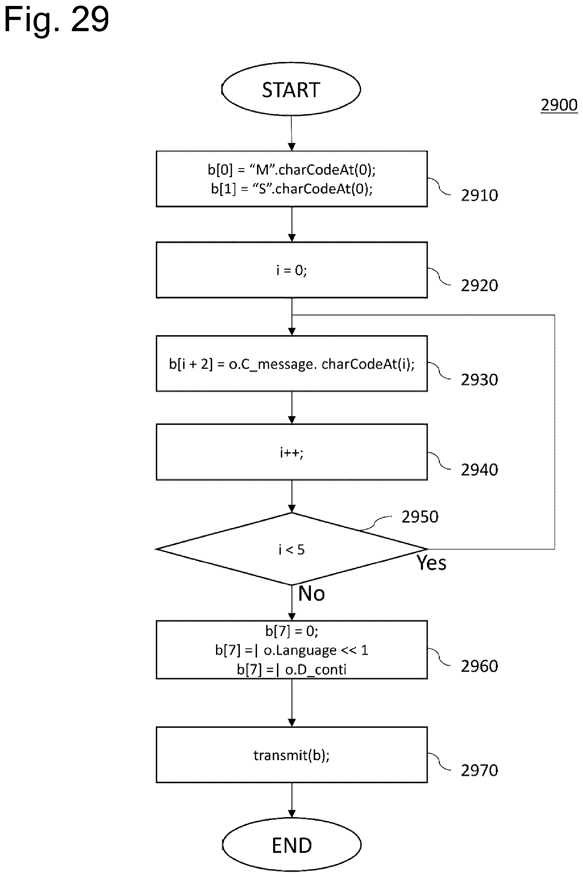

Fig. 29 is a flowchart of a message display request conversion process.

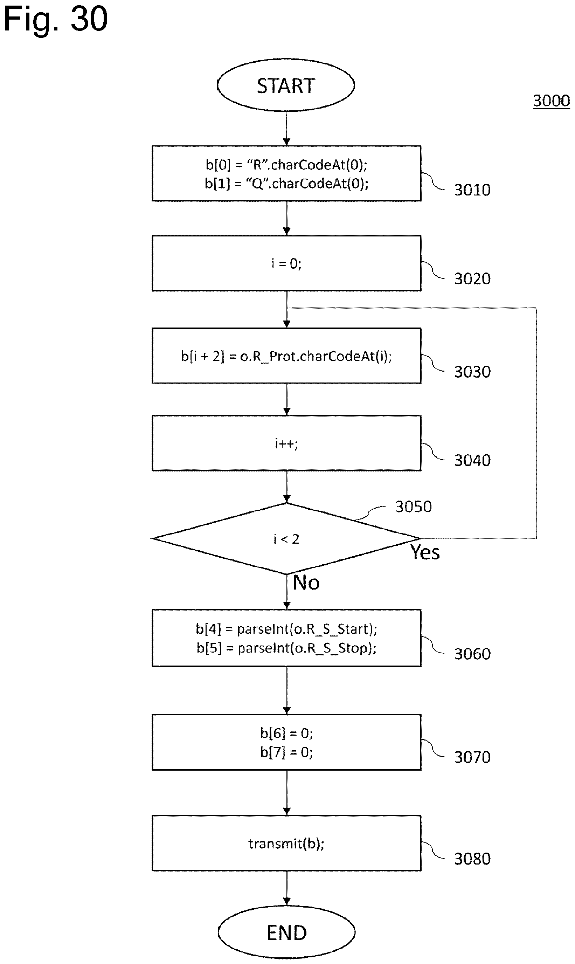

Fig. 30 is a flowchart of a retransmission request conversion process.

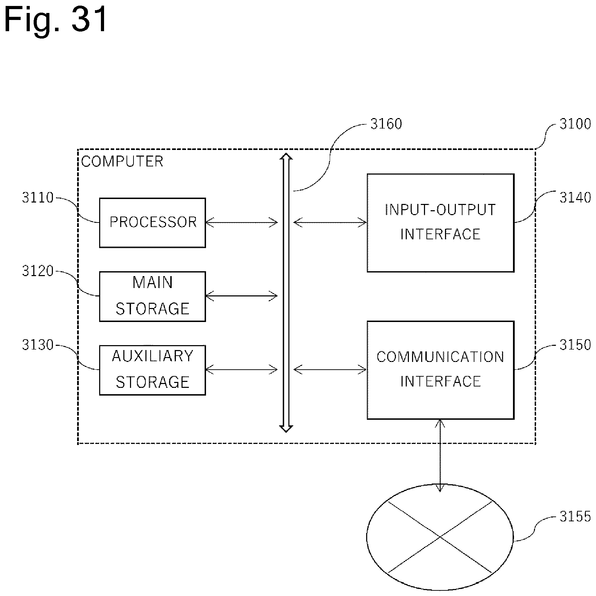

Fig. 31 is a diagram illustrating an example of a hardware configuration of a computer.

### DESCRIPTION OF EMBODIMENTS

### 1 Overview of flavor inhaler or the like according to embodiment of present invention

A flavor inhaler or the like is a device that generates a substance to be inhaled by a user. Hereinafter, an aerosol is described as being the substance generated by the flavor inhaler or the like. Otherwise, the substance generated by the flavor inhaler or the like may also be a gas that is not an aerosol.

### 1-1 First configuration example

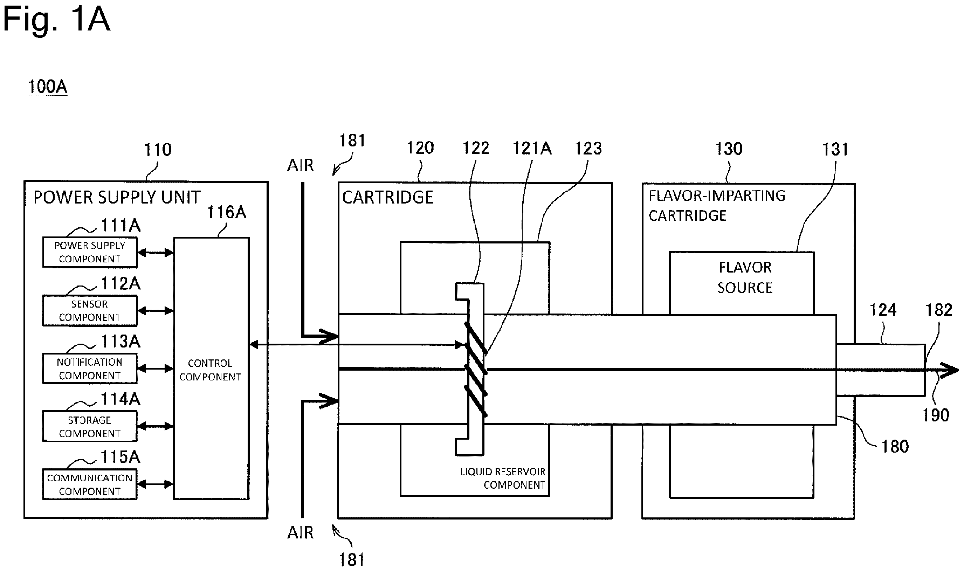

Fig. 1A is a diagram schematically illustrating a first configuration example of the flavor inhaler or the like. As illustrated in Fig. 1A, a flavor inhaler or the like 100A according to the present configuration example includes a power supply unit 110, a cartridge 120, and a flavor-imparting cartridge 130. The power supply unit 110 includes a power supply component 111A, a sensor component 112A, a notification component 113A, a storage component 114A, a communication component 115A, and a control component 116A. The cartridge 120 includes a heating component 121A, a liquid channeling component 122, and a liquid reservoir component 123. The flavor-imparting cartridge 130 includes a flavor source 131 and a mouthpiece 124. An air channel 180 is formed in the cartridge 120 and the flavor-imparting cartridge 130.

Note that the cartridge 120 and the flavor-imparting cartridge 130 are an example of a "refill" described later. In the present embodiment, a color according to the type of refill is applied to at least a part of the refills 120 and/or 130. Also, what is colored according to type is not limited to refills, and may be any component mounted in the flavor inhaler or the like 100A.

The power supply component 111A stores electric power. Additionally, the power supply component 111A supplies electric power to each component of the flavor inhaler or the like 100A on the basis of control by the control component 116A. The power supply component 111A may be formed from a rechargeable battery such as a lithium-ion secondary cell, for example.

The sensor component 112A obtains various information related to the flavor inhaler or the like 100A. The sensor component 112A includes a color sensor for sensing the color according to the type of the component applied to a component (including a refill) mounted in the flavor inhaler or the like 100A. The color sensor may include a white LED and a sensor capable of sensing the intensity of each of the three primary colors RGB. Alternatively, the color sensor may include an LED capable of emitting light in each of the three primary colors RGB individually and a photosensor capable of sensing the intensity of the light. Also, the sensor component 112A includes a sensor capable of sensing the replacement or mounting (hereinafter referred to as "replacement or the like") of a component. Such a sensor may include a contact sensor capable of sensing the replacement or the like of a component. Alternatively, the flavor inhaler or the like 100A may sense the replacement or the like of a component when a predetermined color is sensed using the configuration for sensing a color according to the type of component applied to a component. The sensor component 112A may include a pressure sensor such as a microphone condenser, a flow sensor, a temperature sensor, or the like, and obtains a value associated with inhalation by the user. The sensor component 112A may also include an input device such as a button or a switch for receiving the input of information from the user.

The notification component 113A notifies the user of information. The notification component 113A in the present embodiment includes a display device that displays messages. For example, the notification component 113A may include a light-emitting device that emits light, a display device that displays images, a sound output device that outputs sound, a vibration device that vibrates, or the like.

The storage component 114A stores various information for operations by the flavor inhaler or the like 100A. For example, the storage component 114A is formed from a non-volatile storage medium such as a flash memory. The storage component 114A may also include a volatile memory that provides a work area for control by the control component 116A.

The communication component 115A includes a communication interface (including a communication module) conforming to a predetermined LPWA wireless communication standard or a wireless communication standard with similar limitations. Sigfox, LoRa-WAN, or the like may be adopted as the communication standard. The communication component 115A may also be a communication interface capable of performing communication conforming to any given wired or wireless communication standard. For example, Wi-Fi(R), Bluetooth(R), or the like may be adopted as the communication standard.

The control component 116A functions as a computational processing device and control device, and controls overall operations inside the flavor inhaler or the like 100A by following various programs. The control component 116A is achieved by an electronic circuit such as a central processing unit (CPU) or a microprocessor, for example.

The liquid reservoir component 123 stores an aerosol source. An aerosol is generated by atomizing the aerosol source. For example, the aerosol source may be a polyhydric alcohol such as glycerin or propylene glycol and a liquid such as water. The aerosol source may also contain tobacco-derived or non-tobacco-derived flavor components. In the case where the flavor inhaler or the like 100A is a medical inhaler such as a nebulizer, the aerosol source may also contain a medicine.

The aerosol source, that is, the liquid stored in the liquid reservoir component 123, is channeled from the liquid reservoir component 123 and held by the liquid channeling component 122. For example, the liquid channeling component 122 is a wick formed by twisting fiber materials such as glass fiber or other porous materials such as porous ceramics. In this case, the aerosol source stored in the liquid reservoir component 123 is channeled by the capillary effect of the wick.

The heating component 121A heats the aerosol source to atomize the aerosol source and generate an aerosol. In the example illustrated in Fig. 1A, the heating component 121A is configured as a coil that is wound around the liquid channeling component 122. When the heating component 121A generates heat, the aerosol source held in the liquid channeling component 122 is heated and atomized, and an aerosol is generated. The heating component 121A generates heat when supplied with power from the power supply component 111A. As an example, power may be supplied when the sensor component 112A detects the user starting inhaling and/or the inputting of predetermined information. Additionally, the supply of power may be stopped when the sensor component 112A detects the user finishing inhaling and/or the inputting of predetermined information.

The flavor source 131 is a component for imparting a flavor component to the aerosol. The flavor source 131 may contain tobacco-derived or non-tobacco-derived flavor components.

The air channel 180 is a channel for air to be inhaled by the user. The air channel 180 has a tubular structure with an air inflow hole 181 serving as an inlet for air into the air channel 180 and an air outflow hole 182 serving as an outlet for air from the air channel 180 on either end. Partway through the air channel 180, the liquid channeling component 122 is disposed on the upstream side (the side close to the air inflow hole 181) and the flavor source 131 is disposed on the downstream side (the side close to the air outflow hole 182). Air flowing in from the air inflow hole 181 in association with inhalation by the user is mixed with the aerosol generated by the heating component 121A, and as indicated by an arrow 190, passes through the flavor source 131 and is transported to the air outflow hole 182. When the fluid mixture of aerosol and air passes through the flavor source 131, the flavor component contained in the flavor source 131 is imparted to the aerosol.

The mouthpiece 124 is a member that is put into the user's mouth during inhalation. The air outflow hole 182 is disposed in the mouthpiece 124. The user puts the mouthpiece 124 into the user's mouth and inhales, and can thereby draw in the fluid mixture of aerosol and air into the oral cavity.

The above describes a configuration example of the flavor inhaler or the like 100A. Obviously, the configuration of the flavor inhaler or the like 100A is not limited to the above and may take a variety of configurations exemplified below.

As one example, the flavor inhaler or the like 100A does not have to include the flavor-imparting cartridge 130. In this case, the mouthpiece 124 is provided on the cartridge 120.

As another example, the flavor inhaler or the like 100A may also include multiple types of aerosol sources. By causing multiple types of aerosols generated from the multiple types of aerosol sources to be mixed inside the air channel 180 and inducing a chemical reaction, still other types of aerosols may be generated.

Additionally, the means for atomizing the aerosol source is not limited to heating by the heating component 121A. For example, the means for atomizing the aerosol source may also be vibration atomization or induction heating.

### 1-2 Second configuration example

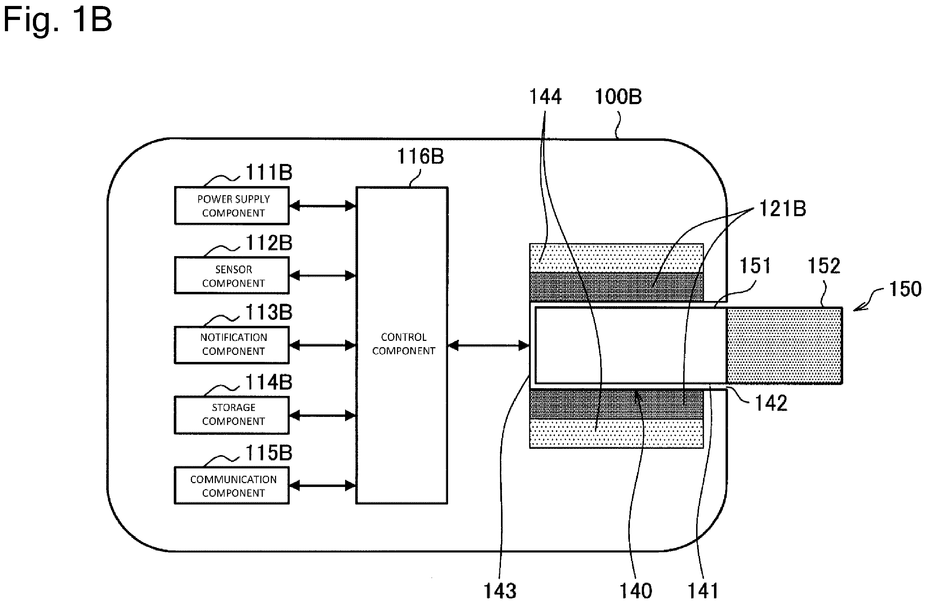

Fig. 1B is a diagram schematically illustrating a second configuration example of the flavor inhaler or the like. As illustrated in Fig. 1B, a flavor inhaler or the like 100B according to the present configuration example includes a power supply component 111B, a sensor component 112B, a notification component 113B, a storage component 114B, a communication component 115B, a control component 116B, a heating component 121B, a holding component 140, and an insulating component 144.

The power supply component 111B, the sensor component 112B, the notification component 113B, the storage component 114B, the communication component 115B, and the control component 116B are each substantially the same as the corresponding component included in the flavor inhaler or the like 100A according to the first configuration example.

The holding component 140 has an internal space 141 and holds a stick-type substrate 150 while accommodating a part of the stick-type substrate 150 in the internal space 141. Note that the stick-type substrate 150 is also an example of a "refill". The holding component 140 has an opening 142 that connects the internal space 141 to the outside and holds the stick-type substrate 150 inserted into the internal space 141 from the opening 142. For example, the holding component 140 is a cylindrical body having the opening 142 and a base component 143 as a base, and demarcates a columnar internal space 141. The holding component 140 also has a function of demarcating a channel for air to be supplied to the stick-type substrate 150. An air inflow hole serving as an inlet for air into the channel is disposed in the base component 143, for example. On the other hand, an air outflow hole serving as an outlet for air from the channel is the opening 142.

The stick-type substrate 150 includes a substrate component 151 and an inhaling component 152. The substrate component 151 includes an aerosol source. Note that in the present configuration example, the aerosol source is not limited to a liquid and may also be a solid. In the state with the stick-type substrate 150 held by the holding component 140, at least a part of the substrate component 151 is accommodated in the internal space 141, and at least a part of the inhaling component 152 projects out from the opening 142. Additionally, if the user puts the inhaling component 152 projecting out from the opening 142 into the user's mouth and inhales, air flows into the internal space 141 from an air inflow hole not illustrated and arrives inside the user's mouth together with an aerosol produced from the substrate component 151.

The heating component 121B has a configuration similar to the heating component 121A according to the first configuration example. However, in the example illustrated in Fig. 1B, the heating component 121B is configured in a film form and is disposed to cover the outer circumference of the holding component 140. Additionally, when the heating component 121B generates heat, the substrate component 151 of the stick-type substrate 150 is heated from the outer circumference, and an aerosol is generated.

The insulating component 144 prevents heat transfer from the heating component 121B to other components. For example, the insulating component 144 is formed from a vacuum insulation material, an aerogel insulation material, or the like.

The above describes a configuration example of the flavor inhaler or the like 100B. Obviously, the configuration of the flavor inhaler or the like 100B is not limited to the above and may take a variety of configurations exemplified below.

As an example, the heating component 121B may be configured in a blade form and may be disposed to project into the internal space 141 from the base component 143 of the holding component 140. In this case, the blade-shaped heating component 121B is inserted into the substrate component 151 of the stick-type substrate 150 and heats the substrate component 151 of the stick-type substrate 150 from the inside. As another example, the heating component 121B may be disposed to cover the base component 143 of the holding component 140. Moreover, the heating component 121B may also be configured as a combination of two or more of a first heating component that covers the outer circumference of the holding component 140, a blade-shaped second heating component, and a third heating component that covers the base component 143 of the holding component 140.

As another example, the holding component 140 may also include an opening-closing mechanism such as a hinge that opens and closes a part of a shell that forms the internal space 141. Additionally, by opening and closing the shell, the holding component 140 may grip the stick-type substrate 150 inserted into the internal space 141. In this case, the heating component 121B may be provided on the gripping component of the holding component 140 and heat the stick-type substrate 150 while pressure is applied.

Additionally, the means for atomizing the aerosol source is not limited to heating by the heating component 121B. For example, the means for atomizing the aerosol source may also be induction heating.

Also, the flavor inhaler or the like 100B may further include the heating component 121A, the liquid channeling component 122, the liquid reservoir component 123, and the air channel 180 according to the first configuration example, and the air outflow hole 182 of the air channel 180 may double as an air inflow hole into the internal space 141. In this case, the fluid mixture of air and an aerosol generated by the heating component 121A flows into the internal space 141, is further mixed with an aerosol generated by the heating component 121B, and arrives inside the user's oral cavity.

### 2 Overview of system according to embodiment of present invention

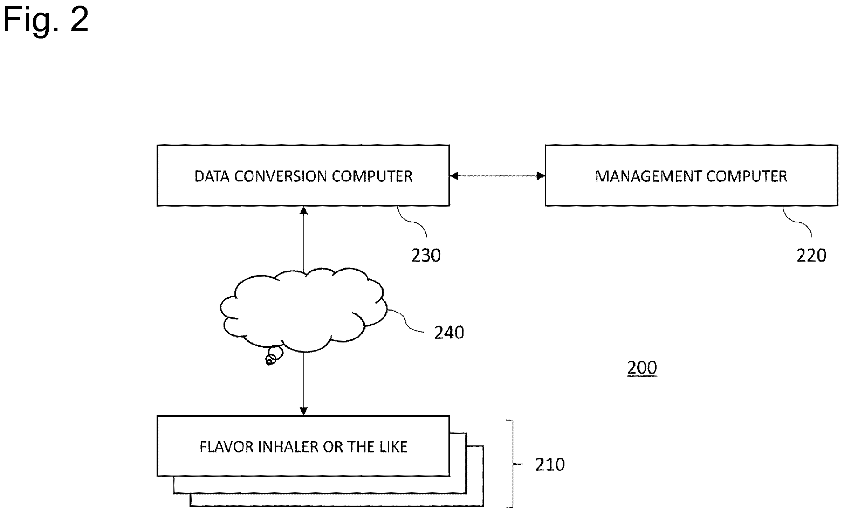

Fig. 2 is a diagram schematically illustrating a configuration example of a system 200 according to one embodiment of the present invention.

Reference numeral 210 denotes the flavor inhaler or the like, such as 100A or 100B, for example. A plurality (possibly a very large number) of flavor inhalers or the like 210 exist. As described above, in the present embodiment, the flavor inhaler or the like 210 includes a communication module conforming to a predetermined LPWA wireless communication standard. The following description presumes Sigfox as an example of the predetermined LPWA wireless communication standard, but it should be understood that the present invention may also be carried out in relation to any communication standard with similar limitations.

Reference numeral 220 denotes a management computer 220 for communicating with the flavor inhaler or the like 210 and managing the flavor inhaler or the like 210. Management of the flavor inhaler or the like 210 includes control of the flavor inhaler or the like 210. The management computer 220 may be formed from a plurality of computers, and may also be a collection of computers with various functions, such as a gateway, a database, and a web server.

Reference numeral 230 denotes a data conversion computer 230 that receives and converts data generated and transmitted by the flavor inhaler or the like 210 for communication with the management computer 220, and then transmits the converted data to the management computer 220, and also receives and converts data generated and transmitted by the management computer 220 for communication with the flavor inhaler or the like 210, and then transmits the converted data to the flavor inhaler or the like 210. Note that the data conversion computer 230 may also be referred to as a "binary parser" in Sigfox. In addition, the data conversion computer 230 may be formed from a plurality of computers.

Reference numeral 240 denotes an LPWA network including a communication channel between the flavor inhaler or the like 210 and the data conversion computer 230. The LPWA network 240 includes what is called the Sigfox cloud.

Note that the flavor inhaler or the like 210 and the data conversion computer 230 may transmit and receive data through one or more communication devices or computers, and the management computer 220 and the data conversion computer 230 may transmit and receive data through one or more communication devices or computers. Also, one or more networks, including the Internet not illustrated, may exist in the communication channel between the flavor inhaler or the like 210 and the data conversion computer 230 and/or the communication channel between the management computer 220 and the data conversion computer 230.

The presumed communication standard has limitations whereby the data that can be transmitted by the flavor inhaler or the like 210 is 12 bytes of binary data per transmission and the data that can be received is 8 bytes of binary data per transmission, for example. Consequently, in the presumed communication standard, when the flavor inhaler or the like 210 and the management computer 220 communicate, binary data transmitted from the flavor inhaler or the like 210 is converted into easily handled JSON data in the data conversion computer 230 and transmitted to the management computer 220, whereas JSON data is transmitted from the data conversion computer 230, converted into binary data in the data conversion computer 230, and transmitted to the flavor inhaler or the like 210. Note that the limitations on the size of binary data that can transmitted and received may differ somewhat depending on the communication standard, but share a common feature in that the data size is small.

Also, in presumed communication standard, communication from a single flavor inhaler or the like 210 to the management computer 220 (hereinafter, this direction of communication is referred to as "upstream") is limited to a maximum of 140 transmissions per day, and communication from the management computer 220 to a single flavor inhaler or the like 210 (hereinafter, this direction of communication is referred to as "downstream") is limited to a maximum of 4 transmissions per day. Furthermore, the presumed communication standard has a limitation whereby the management computer 220 is unable to spontaneously initiate communication.

To address limitations like the above, in the present embodiment, the flavor inhaler or the like 210, the management computer 220, and the data conversion computer 230 execute processes as described below using data as described below.

### 3 Overview of data according to embodiment of present invention

### 3-1 Upstream data

Hereinafter, the binary data that is generated and transmitted by the flavor inhaler or the like 210 and received by the data conversion computer 230 and the JSON data that is generated and transmitted by the data conversion computer 230 in response to the reception and received by the management computer 220 in one embodiment will be described. It should be noted that JSON data is text data, and the actual JSON data generated is a string in a format like the following, for example.

```
{
                          Name 1: "OOO",
                          Name 2: OOO, ...
                  }
```

Here, "Name 1" and "OOO" correspond to a single element, the former being the name of the element and the latter being the value of the element. The same is also true of "Name 2" and "OOO".

### 3-1-1 Data for initial communication

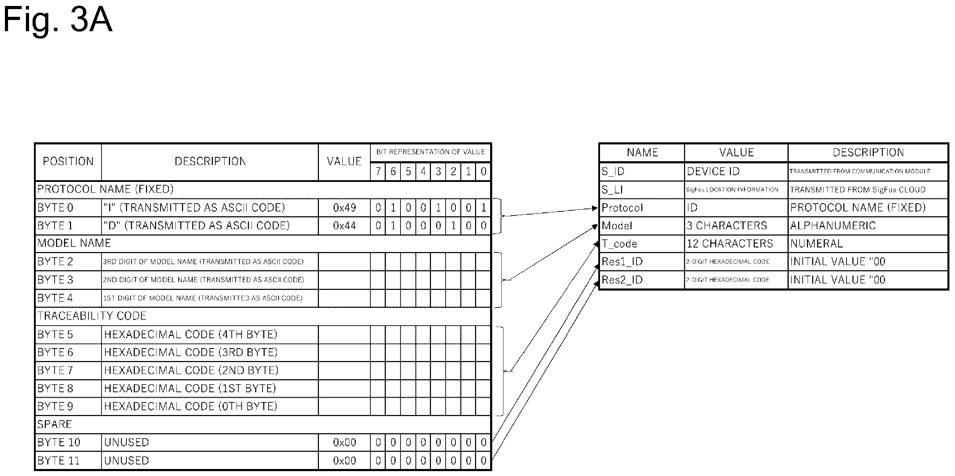

Fig. 3A illustrates the formats of binary data (left) for initial communication that is generated and transmitted by the flavor inhaler or the like 210 and received by the data conversion computer 230, and JSON data (right) for initial communication that is generated through conversion of the binary data and transmitted by the data conversion computer 230 and received by the management computer 220.

In bytes 0 and 1 of the binary data for initial communication, predetermined values (for example, 0x49 and 0x44, respectively) which indicate that the binary data is for initial communication can be set. Note that "0xOO" is a notation denoting the numerical value OO expressed in hexadecimal.

In bytes 2 to 4 of the binary data for initial communication, a value expressing the model of the flavor inhaler or the like 210 that generates the binary data can be set. The value expressing the model may be a three-character model name, and the ASCII code of each character may be set in each byte of bytes 2 to 4 of the binary data.

In bytes 5 to 9 of the binary data for initial communication, a value for identifying the individual flavor inhaler or the like 210 that generates the binary data can be set. The value for identifying the individual flavor inhaler or the like 210 may be a 12-digit numeral (hereinafter referred to as a "traceability code") expressed in decimal, of which the 1st to 7th digits are a serial code, the 8th digit is a line code, the 9th and 10th digits are a color code, and the 11th and 12th digits are a country code, and a numeral for every two digits of the traceability code expressed in hexadecimal can be set as a numerical value in each of bytes 5 to 9 of the binary data. For example, the traceability code "123456789012" can be expressed as "1cbe991a14" in hexadecimal, and 0x1c, 0xbe, 0x99, 0x1a, and 0x14 can be set in bytes 5, 6, 7, 8, and 9, respectively, of the binary data.

Bytes 10 and 11 of the binary data for initial communication are not used in the present embodiment, and a predetermined value (for example, 0x00) can be set in the bytes.

In S_ID and S_LI elements of the JSON data for initial communication, predetermined values based on the presumed communication standard can be set. The same is also true of JSON data for normal communication, JSON data for emergency communication, JSON data for the acknowledgment of various settings request, and JSON data for the acknowledgment of a message display request to be described hereinafter.

In a Protocol element of the JSON data for initial communication, a value corresponding to bytes 0 and 1 of the binary data for initial communication (for example, the corresponding two characters "ID" when 0x49 and 0x44 are interpreted as ASCII codes) can be set.

In a Model element of the JSON data for initial communication, a value corresponding to bytes 2 to 4 of the binary data for initial communication (for example, the corresponding three characters when the values of the bytes are interpreted as ASCII codes) can be set.

In a T_code element of the JSON data for initial communication, a value corresponding to bytes 5 to 9 of the binary data for initial communication (for example, the traceability code which is a 12-digit numeral expressed in decimal) can be set.

In Res1_ID and Res2_ID elements of the JSON data for initial communication, values corresponding to bytes 10 and 11 of the binary data for initial communication can be set. However, since bytes 10 and 11 of the binary data for initial communication are not used in the present embodiment, a predetermined value (for example, the numeral "00") may simply be set in the Res1 ID and Res2_ID elements of the JSON data for initial communication.

### 3-1-2 Data for normal communication

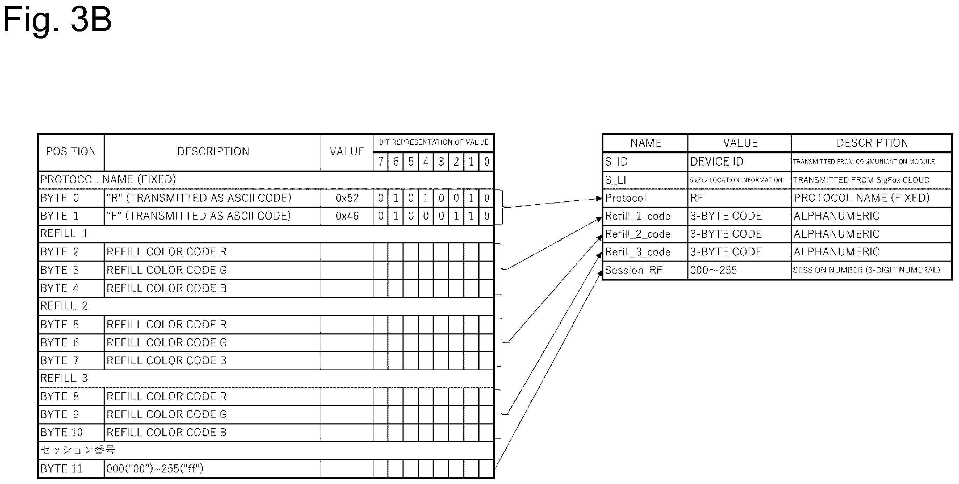

Fig. 3B illustrates the formats of binary data (left) for normal communication that is generated and transmitted by the flavor inhaler or the like 210 and received by the data conversion computer 230, and JSON data (right) for normal communication that is generated through conversion of the binary data and transmitted by the data conversion computer 230 and received by the management computer 220.

In bytes 0 and 1 of the binary data for normal communication, predetermined values (for example, 0x52 and 0x46, respectively) which indicate that the binary data is for normal communication can be set.

A value based on a color code identifying the color of a refill that the flavor inhaler or the like 210 obtains with a sensor can be set in bytes 2 to 4, 5 to 7, and 8 to 10, respectively, of the binary data for normal communication. More specifically, a value indicating the magnitude of an R component of the color code can be set in bytes 2, 5, and 8 of the binary data for normal communication, a value indicating the magnitude of a G component can be set in bytes 3, 6, and 9, and a value indicating the magnitude of a B component can be set in bytes 4, 7, and 10. Note that the set plurality of values are not limited to such RGB values and may also be HSV values, for example. It should be noted that a "refill" is an example of a component that is mounted in the flavor inhaler or the like 210.

In byte 11 of the binary data for normal communication, a session number generated for normal communication can be set.

In a Protocol element of the JSON data for normal communication, a value corresponding to bytes 0 and 1 of the binary data for normal communication (for example, the corresponding two characters "RF" when 0x52 and 0x46 are interpreted as ASCII codes) can be set.

In a Refill 1 code element of the JSON data for normal communication, the six characters obtained by concatenating the numeral expressing in hexadecimal the value of byte 2, the numeral expressing in hexadecimal the value of byte 3, and the numeral expressing in hexadecimal the value of byte 4 of the binary data for normal communication can be set. For example, when the values of bytes 2 to 4 of the binary data for normal communication are 0xc7, 0x15, and 0x85, respectively, the string "c71585" can be set in Refill 1 code of the JSON data for normal communication. Note that the above six characters are an example of a "color code". Refill_2_code and Refill_3_code elements of the JSON data for normal communication may be similar to the Refill 1 code element of the JSON data for normal communication except for corresponding to bytes 5 to 7 and 8 to 10 of the binary data for normal communication.

In a Session RF element of the json data for normal communication, the value of byte 11 of the binary data for normal communication can be set as a three-digit numeral.

### 3-1-3 Data for emergency communication

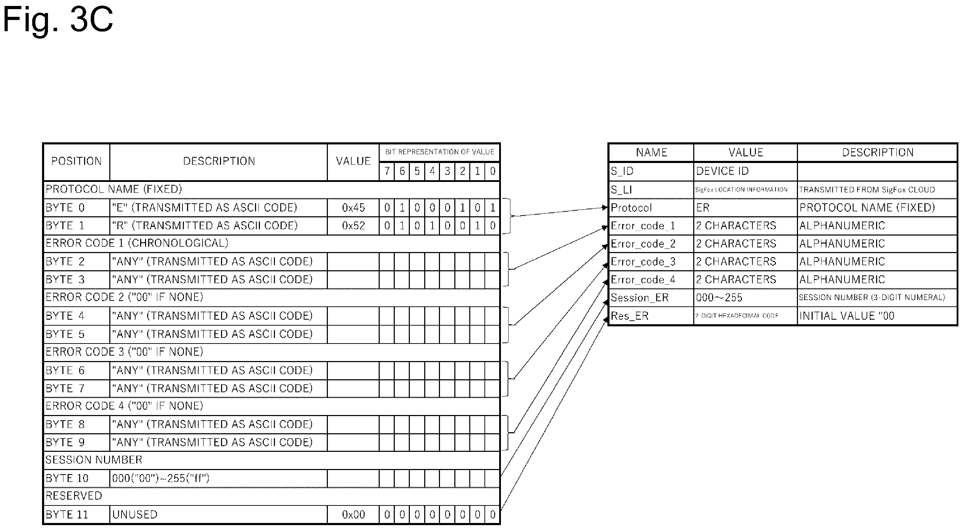

Fig. 3C illustrates the formats of binary data (left) for emergency communication that is generated and transmitted by the flavor inhaler or the like 210 and received by the data conversion computer 230, and JSON data (right) for emergency communication that is generated through conversion of the binary data and transmitted by the data conversion computer 230 and received by the management computer 220.

In bytes 0 and 1 of the binary data for emergency communication, predetermined values (for example, 0x45 and 0x52, respectively) which indicate that the binary data is for emergency communication can be set.

In bytes 2 and 3, 4 and 5, 6 and 7, and 8 and 9, respectively, of the binary data for emergency communication, values based on error codes identifying errors occurring in the flavor inhaler or the like 210 can be set. More specifically, error codes may be two characters, in which the ASCII codes of the first characters of the error codes can be set in bytes 2, 4, 6, and 8 of the binary data for emergency communication and the ASCII codes of the second characters of the error codes can be set in bytes 3, 5, 7, and 8.

In byte 10 of the binary data for emergency communication, a session number generated for emergency communication can be set.

Byte 11 of the binary data for emergency communication is not used in the present embodiment, and a predetermined value (for example, 0x00) can be set in the byte.

In a Protocol element of the JSON data for emergency communication, a value corresponding to bytes 0 and 1 of the binary data for emergency communication (for example, the corresponding two characters "ER" when 0x45 and 0x52 are interpreted as ASCII codes) can be set.

In Error_code_1, Error_code_2, Error_code_3, and Error_code_4 elements of the JSON data for emergency communication, values (for example, two-character error codes) corresponding to bytes 2 and 3, 4 and 5, 6 and 7, and 8 and 9, respectively, of the binary data for emergency communication can be set.

In a Session ER element of the JSON data for emergency communication, the value of byte 10 of the binary data for emergency communication can be set as a three-digit numeral.

In a Res ER element of the JSON data for emergency communication, a value corresponding to byte 11 of the binary data for emergency communication can be set. However, since byte 11 of the binary data for emergency communication is not used in the present embodiment, a predetermined value (for example, the numeral "00") may simply be set in the Res ER element of the JSON data for emergency communication.

### 3-1-4 Data for acknowledgment of various settings request

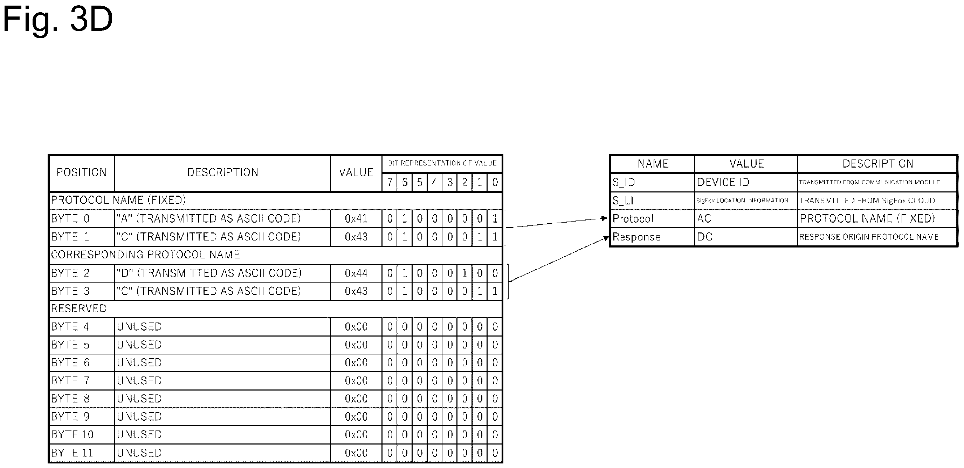

Fig. 3D illustrates the formats of binary data (left) for the acknowledgment of various settings request that is generated and transmitted by the flavor inhaler or the like 210 and received by the data conversion computer 230, and JSON data (right) for the acknowledgment of various settings request that is generated through conversion of the binary data and transmitted by the data conversion computer 230 and received by the management computer 220.

In bytes 0 and 1 of the binary data for the acknowledgment of various settings request, predetermined values (for example, 0x41 and 0x43, respectively) which indicate that the binary data is for acknowledgment can be set.

In bytes 2 and 3 of the binary data for the acknowledgment of various settings request, predetermined values (for example, 0x44 and 0x43, respectively) which indicate that an acknowledgment made using the binary data is in response to a request for various settings can be set.

Bytes 4 to 11 of the binary data for the acknowledgment of various settings request are not used in the present embodiment, and a predetermined value (for example, 0x00) can be set in the bytes.

In a Protocol element of the JSON data for the acknowledgment of various settings request, a value corresponding to bytes 0 and 1 of the binary data for the acknowledgment of various settings request (for example, the corresponding two characters "AC" when 0x41 and 0x43 are interpreted as ASCII codes) can be set.

In a Response element of the JSON data for the acknowledgment of various settings request, a value corresponding to bytes 2 and 3 of the binary data for the acknowledgment of various settings request (for example, the corresponding two characters "DC" when 0x44 and 0x43 are interpreted as ASCII codes) can be set.

### 3-1-5 Data for acknowledgment of message display request

Fig. 3E illustrates the formats of binary data (left) for the acknowledgment of a message display request that is generated and transmitted by the flavor inhaler or the like 210 and received by the data conversion computer 230, and JSON data (right) for the acknowledgment of a message display request that is generated through conversion of the binary data and transmitted by the data conversion computer 230 and received by the management computer 220.

In bytes 0 and 1 of the binary data for the acknowledgment of a message display request, predetermined values (for example, 0x41 and 0x43, respectively) which indicate that the binary data is for acknowledgment can be set.

In bytes 2 and 3 of the binary data for the acknowledgment of a message display request, predetermined values (for example, 0x4d and 0x53, respectively) which indicate that an acknowledgment made using the binary data is in response to a request for a message display can be set.

In byte 4 of the binary data for the acknowledgment of a message display request, a value (for example, the numerical value 1 or 2) indicating whether the received request for a message display is the first time or the second time can be set.

Bytes 5 to 11 of the binary data for the acknowledgment of a message display request are not used in the present embodiment, and a predetermined value (for example, 0x00) can be set in the bytes.

In a Protocol element of the JSON data for the acknowledgment of a message display request, a value corresponding to bytes 0 and 1 of the binary data for the acknowledgment of a message display request (for example, the corresponding two characters "AC" when 0x41 and 0x43 are interpreted as ASCII codes) can be set.

In a Response element of the JSON data for the acknowledgment of a message display request, a value corresponding to bytes 2 and 3 of the binary data for the acknowledgment of a message display request (for example, the corresponding two characters "MS" when 0x4d and 0x53 are interpreted as ASCII codes) can be set.

In an MS Num element of the JSON data for the acknowledgment of a message display request, the value of byte 4 of the binary data for the acknowledgment of a message display request can be set.

### 3-2 Downstream data

Hereinafter, the JSON data that is generated and transmitted by the management computer 220 and received by the data conversion computer 230 and the binary data that is generated and transmitted by the data conversion computer 230 in response to the reception and received by the flavor inhaler or the like 210 in one embodiment will be described.

### 3-2-1 Data for request of time setting

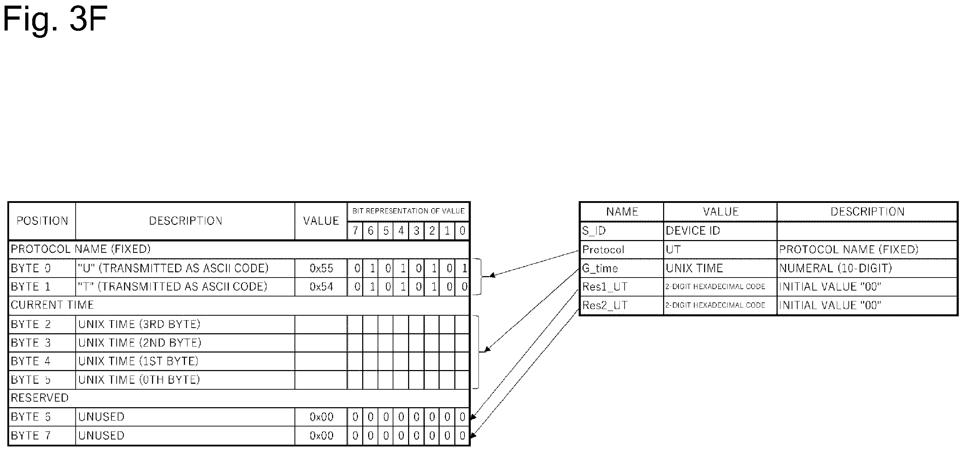

Fig. 3F illustrates the formats of JSON data (right) for the request of time setting that is generated and transmitted by the management computer 220 and received by the data conversion computer 230, and binary data (left) for the request of time setting that is generated through conversion of the JSON data and transmitted by the data conversion computer 230 and received by the flavor inhaler or the like 210.

In an S_ID element of the JSON data for the request of time setting, a predetermined value based on the presumed communication standard can be set. The same is also true of JSON data for the request of various settings, JSON data for the request of a message display, JSON data for requesting the retransmission of refill data, and JSON data for requesting the transmission of error data to be described hereinafter.

In a Protocol element of the JSON data for the request of time setting, a predetermined value (for example, the two characters "UT") indicating that the JSON data is for the request of time setting can be set.

In a G time element of the JSON data for the request of time setting, a 10-digit numeral expressing the time to be set as a decimal UNIX(R) time can be set. For example, 2020/01/15 11:42:13 JST can be expressed as the UNIX(R) time "1579056133".

Res1_UT and Res2UT elements of the JSON data for the request of time setting are not used in the present embodiment, and a predetermined value (for example, the numeral "00") can be set in the elements.

In bytes 0 and 1 of the binary data for time setting, values corresponding to the Protocol element of the JSON data for the request of time setting (for example, the respective ASCII codes 0x55 and 0x54 of the two characters "UT") can be set.

In bytes 2 to 5 of the binary data for time setting, a numeral for every two digits of the G_time element of the JSON data for the request of time setting expressed in hexadecimal can be set as a numerical value. For example, the numeral "1579056133" can be expressed as "5ele7c05" in hexadecimal, and 0x5e, 0x1e, 0x7c, and 0x05 can be set in bytes 2, 3, 4, and 5, respectively, of the binary data.

In bytes 6 and 7 of the binary data for the request of time setting, the values of Res1_UT1 and Res2_UT elements, respectively, of the JSON data for the request of time setting can be set. However, since the Res1_UT1 and Res2_UT elements of the JSON data for the request of time setting are not used in the present embodiment, a predetermined value (for example, 0x00) may simply be set in bytes 6 and 7 of the binary data for the request of time setting.

### 3-2-2 Data for request of various settings

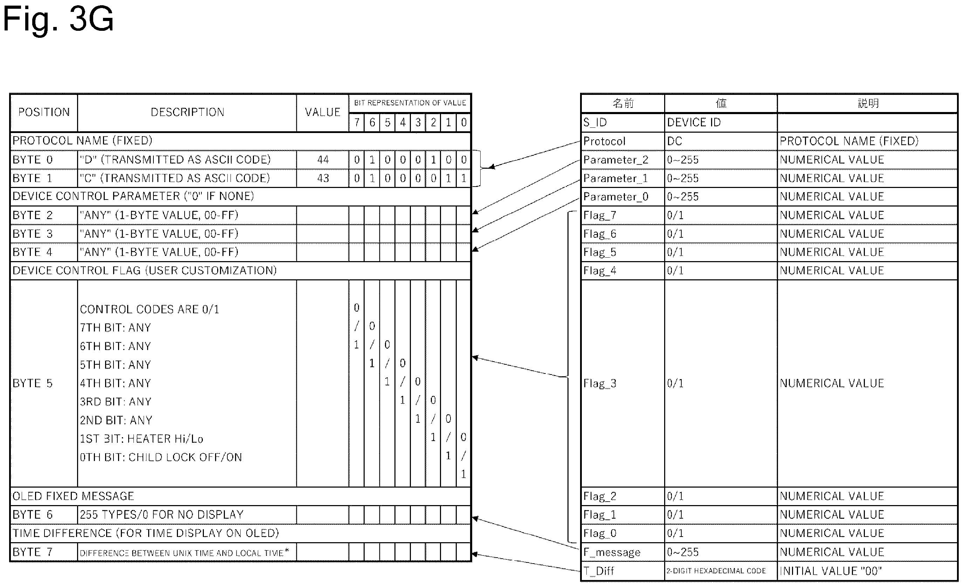

Fig. 3G illustrates the formats of JSON data (right) for the request of various settings that is generated and transmitted by the management computer 220 and received by the data conversion computer 230, and JSON data (left) for the request of various settings that is generated through conversion of the JSON data and transmitted by the data conversion computer 230 and received by the flavor inhaler or the like 210.

In a Protocol element of the JSON data for the request of various settings, a predetermined value (for example, the two characters "DC") indicating that the JSON data is for the request of various settings can be set.

In Parameter_2 to Parameter 0 elements of the JSON data for the request of various settings, a numerical value (0 to 255) of predetermined parameters to be set can be set.

In Flag_7 to Flag_0 elements of the JSON data for the request of various settings, a numerical value (0 or 1) of predetermined flags to be set can be set.

In an F_message element of the JSON data for the request of various settings, a value (0 to 255) specifying a fixed message to be displayed on the flavor inhaler or the like 210 can be set.

In a T Diff element of the JSON data for the request of various settings, a two-digit numeral expressing in hexadecimal a time difference to be reported to the flavor inhaler or the like 210 can be set. For example, in the T Diff element of the JSON data for the request of various settings, a two-digit numeral can be set in which the first digit is 0 in the case where the time difference is + or 1 in the case where the time difference is - with respect to Coordinate Universal Time (UTC), and the second digit is the magnitude of the time difference.

In bytes 0 and 1 of the binary data for the request of various settings, values corresponding to the Protocol element of the JSON data for the request of various settings (for example, the respective ASCII codes 0x44 and 0x43 of the two characters "DC") can be set.

In bytes 2 to 4 of the binary data for the request of various settings, the values of the Parameter 2 to Parameter 0 elements, respectively, of the JSON data for the request of various settings can be set.

In byte 5 of the binary data for the request of various settings, a value based on the values of Flag_7 to Flag_0 of the JSON data for the request of various settings can be set. More specifically, in each of the 7th to 0th bits of byte 5 of the binary data for the request of various settings, the value (0 or 1) of Flag_7 to Flag_0 of the JSON data for the request of various settings can be set.

In byte 6 of the binary data for the request of various settings, the value of the F_message element of the JSON data for the request of various settings can be set.

In byte 7 of the binary data for the request of various settings, the value of the T Diff element of the JSON data for the request of various settings can be set as a numerical value. For example, when the value (two-digit numeral expressed in hexadecimal) of the T Diff element of the JSON data for the request of various settings is "1b", the numerical value 27 can be set in byte 7 of the binary data for the request of various settings.

### 3-2-3 Data for request of message display

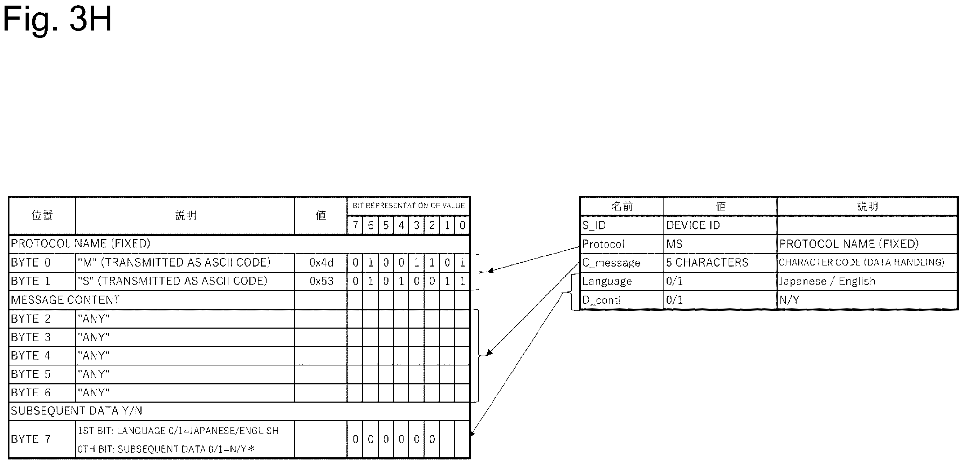

Fig. 3H illustrates the formats of JSON data (right) for the request of a message display on the flavor inhaler or the like 210 that is generated and transmitted by the management computer 220 and received by the data conversion computer 230, and JSON data (left) for the request of a message display that is generated through conversion of the JSON data and transmitted by the data conversion computer 230 and received by the flavor inhaler or the like 210.

In a Protocol element of the JSON data for the request of a message display, a predetermined value (for example, the two characters "MS") indicating that the JSON data is for the request of a message display can be set.

In a C_message element of the JSON data for the request of a message display, the string of a message requested to be displayed can be set.

In a Language element of the JSON data for the request of a message display, the numerical value 1 can be set if the message requested to be displayed is in Japanese or the numerical value 0 can be set if the message requested to be displayed is in English.

In a D_conti element of the JSON data for the request of a message display, the numerical value 1 can be set if there is a continuation of the message requested to be displayed or the numerical value 0 can be set if not.

In bytes 0 and 1 of the binary data for the request of a message display, values corresponding to the Protocol element of the JSON data for the request of a message display (for example, the respective ASCII codes 0x4d and 0x53 of the two characters "MS") can be set.

In bytes 2 to 6 of the binary data for the request of a message display, the respective ASCII codes of the first five characters of the C_message element of the JSON data for the request of a message display can be set. In a case such as where the number of characters in the C_message element of the JSON data for the request of a message display is less than five characters, a predetermined value (for example, the ASCII code 0x20 for a space) may be set in the byte of the binary data for the request of a message display for which there is no corresponding character.

In byte 7 of the binary data for the request of a message display, a value based on the values of the Language and D_conti elements of the JSON data for the request of a message display can be set. More specifically, in one bit of byte 7 of the binary data for the request of a message display, the value (0 or 1) of the Language element of the JSON data for the request of a message display can be set, and in the zero bit, the value (0 or 1) of the D_conti element can be set. In bits 7 to 2 of byte 7 of the binary data for the request of a message display, a predetermined value (for example, 0) may be set.

### 3-2-4 Data for requesting retransmission of refill data

Fig. 3I illustrates the formats of JSON data (right) for requesting the retransmission of refill data that is generated and transmitted by the management computer 220 and received by the data conversion computer 230, and JSON data (left) for requesting the retransmission of refill data that is generated through conversion of the JSON data and transmitted by the data conversion computer 230 and received by the flavor inhaler or the like 210. Note that "refill data" in the present embodiment is the binary data for normal communication described above.

In a Protocol element of the JSON data for requesting the retransmission of refill data, a predetermined value (for example, the two characters "RQ") indicating that the JSON data is for requesting the retransmission of refill data can be set.

In an R_Prot element of the JSON data for requesting the retransmission of refill data, a predetermined value (for example, the two characters "RF") indicating that the retransmission request made using the JSON data is for refill data can be set.

In R_S_Start and R_S_Stop elements of JSON data for requesting the retransmission of refill data, a three-digit value (000 to 255) indicating the start and stop, respectively, of a session number included in the refill data requested to be retransmitted can be set. Although described later, since the session number is generated cyclically in the present embodiment, the stop value is not necessarily larger than the start value, but one of either the start or the stop indicates the maximum value of the range of session numbers according to the retransmission request while the other indicates the minimum value.

In bytes 0 and 1 of the binary data for requesting the retransmission of refill data, values corresponding to the Protocol element of the JSON data for requesting the retransmission of refill data (for example, the respective ASCII codes 0x52 and 0x51 of the two characters "RQ") can be set.

In bytes 2 and 3 of the binary data for requesting the retransmission of refill data, values corresponding to the R_Prot element of the JSON data for requesting the retransmission of refill data (for example, the respective ASCII codes 0x52 and 0x46 of the two characters "RF") can be set.

In bytes 4 and 5 of the binary data for requesting the retransmission of refill data, the values of the R S Start and R S_Stop elements, respectively, of the JSON data for requesting the retransmission of refill data can be set as numerical values.

Bytes 6 and 7 of the binary data for requesting the retransmission of refill data are not used in the present embodiment, and a predetermined value (for example, 0x00) can be set in the bytes.

### 3-2-5 Data for requesting retransmission of error data

Fig. 3J illustrates the formats of JSON data (right) for requesting the retransmission of error data that is generated and transmitted by the management computer 220 and received by the data conversion computer 230, and JSON data (left) for requesting the retransmission of error data that is generated through conversion of the JSON data and transmitted by the data conversion computer 230 and received by the flavor inhaler or the like 210. Note that "error data" in the present embodiment is the binary data for emergency communication described above.

In a Protocol element of the JSON data for requesting the retransmission of error data, a predetermined value (for example, the two characters "RQ") indicating that the JSON data is for requesting the retransmission of refill data can be set.

In an R Prot element of the JSON data for requesting the retransmission of error data, a predetermined value (for example, the two characters "ER") indicating that the retransmission request made using the JSON data is for error data can be set.

In R_S_Start and R_S_Stop elements of JSON data for requesting the retransmission of error data, a three-digit value (000 to 255) indicating the start and stop, respectively, of a session number included in the error data requested to be retransmitted can be set.

In bytes 0 and 1 of the binary data for requesting the retransmission of error data, values corresponding to the Protocol element of the JSON data for requesting the retransmission of error data (for example, the respective ASCII codes 0x52 and 0x51 of the two characters "RQ") can be set.

In bytes 2 and 3 of the binary data for requesting the retransmission of error data, values corresponding to the R Prot element of the JSON data for requesting the retransmission of error data (for example, the respective ASCII codes 0x45 and 0x52 of the two characters "ER") can be set.

In bytes 4 and 5 of the binary data for requesting the retransmission of error data, the values of the R_S_Start and R S_Stop elements, respectively, of the JSON data for requesting the retransmission of error data can be set as numerical values.

Bytes 6 and 7 of the binary data for requesting the retransmission of error data are not used in the present embodiment, and a predetermined value (for example, 0x00) can be set in the bytes.

### 4 Overview of processes according to embodiment of present invention

Hereinafter, processes executed in the flavor inhaler or the like 210, the management computer 220, and the data conversion computer 230 will be described. Note that in the following, pseudocode similar to Javascript is used for some processes, but this is for the sake of explanation and the processes may be implemented using any language. Moreover, the logic and algorithms in the following description are merely examples, and it should be noted that any logic and algorithms not departing from the gist of the present invention can be used to carry out the present invention.

### 4-1 Processes executed in flavor inhaler or the like 210

Hereinafter, processes executed in the flavor inhaler or the like 210 in one embodiment will be described.

### 4-1-1 Initial communication process

Fig. 4 is a flowchart of at least a part of a process (hereinafter referred to as an "initial communication process") 400 for performing initial communication with the management computer 220, the process 400 being performed when triggered by the initial power-on or by the pressing of a reset switch of the flavor inhaler or the like 210.

Reference numeral 410 denotes a step for generating the binary data for initial communication.

Reference numeral 420 denotes a step for transmitting the generated binary data to the data conversion computer 230.

It should be noted that the binary data transmitted from the flavor inhaler or the like 210 is transmitted to the management computer 220 after being converted in the data conversion computer 230.

Reference numeral 430 denotes a step for determining if transmission is successful and a response to the initial communication is received from the management computer 220. If a response is received, the process proceeds to step 440, whereas if not, the process proceeds to step 450.

Reference numeral 440 denotes a step for executing a process (hereinafter referred to as a "response analysis process") for analyzing the response received from the management computer 220 (more precisely, the binary data received from the data conversion computer 230). The response analysis process will be described later.

Reference numeral 450 denotes a step for determining whether a transmission retry should be attempted. In this step, to achieve a predetermined number (for example, three) transmission retries, it is determined that a transmission retry should be attempted if step 420 has been executed a number of times equal to or less than the predetermined number. If it is determined that a transmission retry should be attempted, the process returns to step 420, whereas if not, the process ends.

### 4-1-2 Normal communication process

Fig. 5 is a flowchart of at least a part of a process (hereinafter referred to as a "normal communication process") 500 for executing normal communication with the management computer 220, the process 500 being performed when triggered by the detection of a refill replacement or the like in the flavor inhaler or the like 210.

Reference numeral 510 denotes a step for obtaining a color code of a refill from a memory. In one embodiment, when a refill is replaced or the like, the flavor inhaler or the like 210 can be configured to use a sensor to obtain a color code associated with the mounted refill and store the obtained color code in the memory before performing the normal communication process. Also, in one embodiment, a plurality of color codes can be stored in a memory of the flavor inhaler or the like 210, and in step 510, if a plurality of color codes are stored in the memory, up to a predetermined number of (for example, three) color codes can be obtained from the memory in order from oldest to newest storage.

Reference numeral 520 denotes a step for generating the binary data for normal communication on the basis of the obtained color code(s) (equal to or greater than 1 and less than or equal to the predetermined number). As described above, the binary data for normal communication can include a session number, and the above step can include a step for generating a session number for normal communication. Note that the session number can be generated cyclically. This can be achieved by, for example, dividing a number generated as a candidate for the session number by a predetermined number (for example, 256) and treating the remainder as the generated session number. Furthermore, the session number can be generated consecutively. This can be achieved by, for example, treating the last generated session number + 1 as a candidate for the session number to be generated next.

Reference numeral 530 denotes a step for transmitting the generated binary data to the data conversion computer 230.

Reference numeral 540 denotes a step for determining whether the transmission of binary data is unsuccessful. Note that unsuccessful transmission in this step means that for some reason, such as that a base station associated with the LPWA network was not found, for example, a process pertaining to transmission is unsuccessful inside the flavor inhaler or the like 210. If it is determined that transmission is unsuccessful, the process ends, whereas if not, the process proceeds to step 550.

Reference numeral 550 denotes a step for removing the color code(s) obtained in step 510 from the memory.

According to steps 510, 540, and 550, if the transmission of binary data for normal communication based on a certain color code is successful, the color code is removed and therefore is no longer used to generate subsequent binary data for normal communication, whereas if transmission is unsuccessful, the color code is not removed and therefore is used to generate subsequent binary data for normal communication.

Reference numeral 560 denotes a step for storing the binary data for normal communication generated in step 520 as "refill data" in the memory. In one embodiment, the binary data for normal communication, or in other words the refill data, includes a session number, and one set of refill data can be stored for every session number in the memory of the flavor inhaler or the like 210. Here, in the case of attempting to store refill data including a certain session number, if different refill data including the same session number is already stored, the different refill data may be overwritten. This arrangement is one example of a method for preventing a plurality of refill data including the same session number from being stored.

Reference numeral 570 denotes a step for determining if transmission is successful and a response to the normal communication is received from the management computer 220. If a response is received, the process proceeds to step 580, whereas if not, the process ends.

Reference numeral 580 denotes a step for executing a response analysis process.

### 4-1-3 Emergency communication process

Fig. 6 is a flowchart of at least a part of a process (hereinafter referred to as an "emergency communication process") 600 for executing emergency communication with the management computer 220, the process 600 being performed in response to the occurrence of an error in the flavor inhaler or the like 210.

Reference numeral 610 denotes a step for obtaining an error code from a memory. In one embodiment, when an error occurs, the flavor inhaler or the like 210 can be configured to store an error code corresponding to the error in the memory before performing the emergency communication process. Also, in one embodiment, a plurality of error codes can be stored in a memory of the flavor inhaler or the like 210, and in step 610, if a plurality of error codes are stored in the memory, up to a predetermined number of (for example, four) error codes can be obtained from the memory in order from oldest to newest storage.

Reference numeral 620 denotes a step for generating the binary data for emergency communication on the basis of the obtained error code(s) (equal to or greater than 1 and less than or equal to the predetermined number). The binary data for emergency communication can include a session number, and the above step can include a step for generating a session number for the binary data for emergency communication.

Reference numeral 630 denotes a step for transmitting the generated binary data to the data conversion computer 230.

Reference numeral 640 denotes a step for determining whether the transmission of binary data is unsuccessful. Note that unsuccessful transmission in this step means that for some reason, such as that a base station associated with the LPWA network was not found, for example, a process pertaining to transmission is unsuccessful inside the flavor inhaler or the like 210. If it is determined that transmission is unsuccessful, the process ends, whereas if not, the process proceeds to step 650.

Reference numeral 650 denotes a step for removing the error code(s) obtained in step 610 from the memory.

According to steps 610, 640, and 650, if the transmission of binary data for emergency communication based on a certain error code is successful, the error code is removed and therefore is no longer used to generate subsequent binary data for emergency communication, whereas if transmission is unsuccessful, the error code is not removed and therefore is used to generate subsequent binary data for normal communication.

Reference numeral 660 denotes a step for storing the binary data for emergency communication generated in step 620 as "error data" in the memory. In one embodiment, the binary data for emergency communication, or in other words the error data, includes a session number, and an area for storing one set of error data for every session number can be secured in the memory of the flavor inhaler or the like 210. Here, in the case of attempting to store error data having a certain session number, if different error data having the same session number is already stored, the different error data may be overwritten. This arrangement is one example of a method for preventing a plurality of error data having the same session number from being stored.

Reference numeral 670 denotes a step for determining if transmission is successful and a response to the emergency communication is received from the management computer 220. If a response is received, the process proceeds to step 680, whereas if not, the process ends.

Reference numeral 680 denotes a step for executing a response analysis process.

### 4-1-4 Response analysis process

Fig. 7 is a flowchart of at least a part of a response analysis process 700.

Reference numeral 710 denotes a step for determining whether a predetermined first portion of the binary data received from the data conversion computer 230 as a response received from the management computer 220, specifically the values of bytes 0 and 1, are 0x52 and 0x51, respectively (the ASCII codes for the characters "R" and "Q", respectively). If the values of bytes 0 and 1 of the binary data are 0x52 and 0x51, respectively, it is determined that the retransmission of data is requested from the management computer 220 and the process proceeds to step 715, whereas if not, the process proceeds to step 720.

Reference numeral 715 denotes a step for executing a retransmission process. The retransmission process will be described later.

Reference numeral 720 denotes a step for determining whether the values of bytes 0 and 1 of the binary data are 0x44 and 0x43, respectively (the ASCII codes for the characters "D" and "C", respectively). If the values of bytes 0 and 1 of the binary data are 0x44 and 0x43, respectively, it is determined that various settings for data are requested from the management computer 220 and the process proceeds to step 725, whereas if not, the process proceeds to step 730.

Reference numeral 725 denotes a step for executing a various settings process. The various settings process will be described later.

Reference numeral 730 denotes a step for determining whether the values of bytes 0 and 1 of the binary data are 0x4D and 0x53, respectively (the ASCII codes for the characters "M" and "S", respectively). If the values of bytes 0 and 1 of the binary data are 0x4D and 0x53, respectively, it is determined that a message display of data is requested from the management computer 220 and the process proceeds to step 735, whereas if not, it is determined that time setting is requested from the management computer 220 and the process proceeds to step 740.

Reference numeral 735 denotes a step for executing a message display process. The message display process will be described later.

Reference numeral 740 denotes a step for executing a time setting process. The time setting process can include a step for interpreting a second portion of the binary data, specifically bytes 2 to 5, as data expressing a UNIX(R) time.

### 4-1-5 Retransmission process

Fig. 8 is a flowchart of at least a part of a retransmission process 800.

Reference numeral 810 denotes a step for determining whether there is a data transmission on standby in the flavor inhaler or the like 210. If it is determined that there is a data transmission on standby, the process proceeds to step 815, whereas if not, the process proceeds to step 820.

Reference numeral 815 denotes a step for deferring a data transmission on standby. Note that the deferred data transmission on standby is deferred until a predetermined process (for example, step 850 in Fig. 8) is completed, and after the predetermined process is completed, the deferred data transmission on standby is executed in step 860 described later.

Reference numeral 820 denotes a step for determining whether bytes 2 and 3 of the binary data are 0x52 and 0x46, respectively (the ASCII codes for the characters "R" and "F", respectively). If the values of bytes 0 and 1 of the binary data are 0x52 and 0x46, respectively, it is determined that the retransmission of refill data of data is requested from the management computer 220 and the process proceeds to step 825, whereas if not, it is determined that the retransmission of error data is requested from the management computer 220 and the process proceeds to step 830.

Reference numeral 825 denotes a step for obtaining from the memory, on the basis of bytes 4 and 5 of the binary data, the refill data requested to be retransmitted, namely the refill data that is missing in the management computer 220. In the present embodiment, bytes 4 and 5 of the binary data indicate the start and stop points of the range of session numbers generated cyclically and consecutively. Consequently, step 825 may include a step for obtaining, from the memory, a single set of refill data including a session number inside the range.

Reference numeral 830 denotes a step for obtaining from the memory, on the basis of bytes 4 and 5 of the binary data, the error data requested to be retransmitted, namely the error data that is missing in the management computer 220. In one embodiment, bytes 4 and 5 of the binary data may indicate the start and stop points of the range of session numbers generated cyclically and consecutively. Consequently, step 830 may include a step for obtaining, from the memory, a single set of error data including a session number inside the range.

Reference numeral 840 denotes a step for transmitting the obtained missing refill data or error data (hereinafter referred to as the "data" in this section) to the data conversion computer 230.

Reference numeral 850 denotes a step for determining whether there is still data requested to be retransmitted, namely data that is missing in the management computer 220. This step can include a step for determining whether data including the session numbers in the above range has all been transmitted. If it is determined that there is still missing data, the process returns to step 820, whereas if not, the process ends.

Reference numeral 860 denotes a step for executing a deferred data transmission on standby. It should be noted that step 860 does not exist if a data transmission on standby is not deferred. Also, step 860 may be executed as a part of the retransmission process 800, or as a process separate from the minimization process 800.

### 4-1-6 Various settings process

Fig. 9 is a flowchart of at least a part of a various settings process 900.

Reference numeral 910 denotes a step for determining whether there is a data transmission on standby in the flavor inhaler or the like 210. If it is determined that there is a data transmission on standby, the process proceeds to step 915, whereas if not, the process proceeds to step 920.

Reference numeral 915 denotes a step for deferring a data transmission on standby. Note that the deferred data transmission on standby is deferred until a predetermined process (for example, step 950 or 960 in Fig. 9) is completed, and after the predetermined process is completed, the deferred data transmission on standby is executed in step 970 described later.

Reference numeral 920 denotes a step for setting predetermined parameters on the basis of bytes 2 to 4 of the binary data.

Reference numeral 922 denotes a step for setting a flag related to a heater on the basis of bit 1 of byte 5 of the binary data.

Reference numeral 924 denotes a step for setting a flag related to a child lock function on the basis of bit 0 of byte 5 of the binary data.

Reference numeral 926 denotes a step for setting a fixed message to be displayed on the flavor inhaler or the like 210 on the basis of byte 6 of the binary data.

Reference numeral 928 denotes a step for setting a time difference in the flavor inhaler or the like 210 on the basis of byte 7 of the binary data.

Note that the settings in steps 920, 926, and 928 are examples of control based on one or more bytes of data among the binary data in units of bytes, while the settings in steps 922 and 924 are examples of control based on a bit of data among the binary data.

Reference numeral 930 denotes a step for generating binary data for the acknowledgment of various settings request.

Reference numeral 940 denotes a step for transmitting the generated binary data to the data conversion computer 230.

Reference numeral 950 denotes a step for determining if transmission is successful and a response to the acknowledgment of various settings request is received from the management computer 220. If a response is received, the process proceeds to step 960, whereas if not, the process ends.

Reference numeral 960 denotes a step for executing a response analysis process.

Reference numeral 970 denotes a step for executing a deferred data transmission on standby. It should be noted that step 970 does not exist if a data transmission on standby is not deferred. Also, step 970 may be executed as a part of the various settings process 900, or as a process separate from the various settings process 900.

### 4-1-7 Message display process

Fig. 10 is a flowchart of at least a part of a message display process 1000.

Reference numeral 1010 denotes a step for determining whether there is a data transmission on standby in the flavor inhaler or the like 210. If it is determined that there is a data transmission on standby, the process proceeds to step 1015, whereas if not, the process proceeds to step 1020.

Reference numeral 1015 denotes a step for deferring a data transmission on standby. Note that the deferred data transmission on standby is deferred until a predetermined process (for example, step 1060 or 1070 in Fig. 10) is completed, and after the predetermined process is completed, the deferred data transmission on standby is executed in step 1080 described later.

Reference numeral 1020 denotes a step for determining the language of a message to be displayed on the basis of bit 1 of byte 7 of the binary data. Note that this determination is an example of control based on a bit of data among the binary data.

Reference numeral 1030 denotes a step for displaying a message on the flavor inhaler or the like 210 on the basis of bytes 2 to 6 of the binary data. The message display is an example of control based on one or more bytes of data among the binary data in units of bytes.

Reference numeral 1040 denotes a step for generating binary data for the acknowledgment of a message display request.

Reference numeral 1050 denotes a step for transmitting the generated binary data to the data conversion computer 230.

Reference numeral 1060 denotes a step for determining if transmission is successful and a response to the acknowledgment of a message display request is received from the management computer 220. If a response is received, the process proceeds to step 1070, whereas if not, the process ends.

Reference numeral 1070 denotes a step for executing a response analysis process.

Reference numeral 1080 denotes a step for executing a deferred data transmission on standby. It should be noted that step 1080 does not exist if a data transmission on standby is not deferred. Also, step 1080 may be executed as a part of the message display process 1000, or as a process separate from the message display process 1000.

### 4-2 Processes executed in management computer 220

Hereinafter, processes executed in the management computer 220 in one embodiment will be described.

### 4-2-1 Response process

Fig. 11 is a flowchart of at least a part of a response process 1100 performed when triggered by the reception of some kind of JSON data from the data conversion computer 230.

Reference numeral 1110 denotes a step for converting JSON data jsonDATA (text data) received from the data conversion computer 230 into an object ro. Note that the use of an object is for the sake of simplicity in describing the process, and various processes may also be performed as string operations on the JSON data which is text data. Moreover, an element of an object is equivalent to an element of the same name in the JSON data. Consequently, a reference to an "element" of an "object" hereinafter can be read as an "element of JSON data".

Reference numeral 1120 denotes a step for determining whether the value of a Protocol element of the object ro is the string "ID". If the value of the Protocol element of the object ro is the string "ID", it is determined that the JSON data is JSON data for initial communication and the process proceeds to step 1125, whereas if not, the process proceeds to step 1130.

Reference numeral 1125 denotes a step for executing a response process with respect to initial communication (hereinafter referred to as an "initial communication response process"). The initial communication response process will be described later.

Reference numeral 1130 denotes a step for determining whether the value of the Protocol element of the object ro is the string "RF". If the value of the Protocol element of the object ro is the string "RF", it is determined that the JSON data is JSON data for normal communication and the process proceeds to step 1135, whereas if not, the process proceeds to step 1140.

Reference numeral 1135 denotes a step for executing a response process with respect to normal communication or communication according to the refill data retransmission process (hereinafter referred to as a "normal communication or the like response process"). The normal communication or the like response process will be described later.

Reference numeral 1140 denotes a step for determining whether the value of the Protocol element of the object ro is the string "ER". If the value of the Protocol element of the object ro is the string "ER", it is determined that the JSON data is JSON data for emergency communication and the process proceeds to step 1145, whereas if not, it is determined that the JSON data is JSON data for acknowledgment and the process proceeds to step 1150.

Reference numeral 1145 denotes a step for executing a response process with respect to emergency communication or communication according to the error data retransmission process (hereinafter referred to as an "emergency communication or the like response process"). The emergency communication or the like response process will be described later.

Reference numeral 1150 denotes a step for executing a response process with respect to communication of an acknowledgment (hereinafter referred to as an "acknowledgment communication response process"). The acknowledgment communication response process will be described later.

### 4-2-2 Initial communication response process

Fig. 12 is a flowchart of at least a part of an initial communication response process 1200.

Reference numeral 1210 denotes a step for registering, on the basis of at least the value of Mode and T_code elements of the object ro, the flavor inhaler or the like 210 that has performed initial communication.

Reference numeral 1220 denotes a step for setting the string "UT" in the protocol element of an object to be used to generate JSON data for request of time setting as a response to initial communication.

Reference numeral 1230 denotes a step for setting a 10-digit numeral expressing the time to be set as a decimal UNIX(R) time in a G time element of the object to.

Reference numeral 1240 denotes a step for converting the object to into JSON data and transmitting the converted JSON data to the data conversion computer 230 as a response. It should be noted that the JSON data transmitted from the management computer 220 is transmitted to the flavor inhaler or the like 210 after being converted in the data conversion computer 230.

### 4-2-3 Normal communication or the like response process

Fig. 13 is a flowchart of at least a part of a normal communication or the like response process 1300.

Reference numeral 1310 denotes a step for performing a predetermined process based on the values of Refill 1 code, Refill_2_code, and Refill_3_code elements of the object ro. The predetermined process can include a step for storing the value of a Session RF element of the object ro as the session number of received refill data in the memory. The predetermined process can also include a process for identifying the type of refill (hereinafter referred to as a "refill type process"). The refill type identification process will be described later. Furthermore, the predetermined process may include storing the identified type of refill for a desired purpose.

Reference numeral 1320 denotes a step for checking for missing refill data on the basis of the value of the Session RF element of the object ro, and reference numeral 1330 denotes a step for determining whether there is missing refill data. The management computer 220 can determine that there is missing refill data if the value stored as the session number of the last received refill data and the value of the Session RF element of the object ro are not consecutive. If it is determined that there is missing refill data, the process proceeds to step 1335, whereas if not, the process ends.

Reference numeral 1335 denotes a step for executing a process for requesting the retransmission of missing refill data (hereinafter referred to as a "refill data retransmission request process"). The refill data retransmission request process will be described later.

Reference numeral 1340 denotes a step for determining whether various settings should be requested to the flavor inhaler or the like 210. If it is determined that various settings should be requested, the process proceeds to step 1345, whereas if not, the process proceeds to step 1350.

Reference numeral 1345 denotes a step for executing a process for requesting various settings to the flavor inhaler or the like 210 (hereinafter, a "various settings request process"). The various settings request process will be described later.

Reference numeral 1350 denotes a step for determining whether a message display should be requested to the flavor inhaler or the like 210. If it is determined that a message display should be requested, the process proceeds to step 1355, whereas if not, the process ends.

Reference numeral 1355 denotes a step for executing a process for requesting a message display to the flavor inhaler or the like 210 (hereinafter, a "message display request process"). The message display request process will be described later.

### 4-2-4 Emergency communication or the like response process

Fig. 14 is a flowchart of at least a part of an emergency communication or the like response process 1400.

Reference numeral 1410 denotes a step for performing a predetermined process based on the values of Error code 1, Error_code_2, Error_code_3, and Error_code_4 elements of the object ro. The predetermined process can include a step for storing the value of a Session ER element of the object ro as the session number of received error data in the memory.

Reference numeral 1420 denotes a step for checking for missing error data on the basis of the value of the Session ER element of the object ro, and reference numeral 1430 denotes a step for determining whether there is missing error data. The management computer 220 can determine that there is missing error data if the value stored as the session number of the received error data and the value of the Session ER element of the object ro are not consecutive. If it is determined that there is missing error data, the process proceeds to step 1435, whereas if not, the process ends.

Reference numeral 1435 denotes a step for executing a process for requesting the retransmission of missing error data (hereinafter referred to as an "error data retransmission request process"). The error data retransmission request process will be described later.

### 4-2-5 Acknowledgment communication response process

Fig. 15 is a flowchart of at least a part of an acknowledgment communication response process 1500.

Reference numeral 1510 denotes a step for determining whether various settings should be requested to the flavor inhaler or the like 210 that performs the acknowledgment communication. If it is determined that various settings should be requested, the process proceeds to step 1515, whereas if not, the process proceeds to step 1520.

Reference numeral 1515 denotes a step for executing a various settings request process with respect to the flavor inhaler or the like 210 that performs the acknowledgment communication.

Reference numeral 1520 denotes a step for determining whether a message display should be requested to the flavor inhaler or the like 210 that performs the acknowledgment communication. If it is determined that a message display should be requested, the process proceeds to step 1525, whereas if not, the process ends.

Reference numeral 1525 denotes a step for executing a message display request process with respect to the flavor inhaler or the like 210 that performs the acknowledgment communication.

### 4-2-6 Various settings request process

Fig. 16 is a flowchart of at least a part of a various settings request process 1600.

Reference numeral 1610 denotes a step for setting the string "DC" in the protocol element of the object to be used to generate JSON data for the request of various settings.

Reference numeral 1620 denotes a step for setting values to be set in Parameter_0 to Parameter_2 elements of the object to.

Reference numeral 1630 denotes a step for setting values to be set in Flag_0 to Flag_7 elements of the object to.

Reference numeral 1640 denotes a step for setting a value to be set in an F_message element of the object to.

Reference numeral 1650 denotes a step for setting a value to be set in a T diff element of the object to.

Reference numeral 1660 denotes a step for converting the object to into JSON data and transmitting the converted JSON data to the data conversion computer 230 as a response.

Reference numeral 1670 denotes a step for determining whether there is an acknowledgment from the flavor inhaler or the like within a predetermined time, such as 56 seconds, for example, after the execution of step 1660. In step 1670, it may be determined that there is an acknowledgment when step 1150 is executed after the execution of step 1660. If it is determined that there is an acknowledgment, the process ends, whereas if not, the process returns to step 1660.

### 4-2-7 Message display request process

Fig. 17 is a flowchart of at least a part of a message display request process 1700.

Reference numeral 1710 denotes a step for setting the string "MS" in the protocol element of the object to be used to generate JSON data for the request of a message display.

Reference numeral 1720 denotes a step for setting a string expressing at least a part of a message requested to be displayed in a C_message element of the object to.

Reference numeral 1730 denotes a step for setting a value indicating the language of the message requested to be displayed in a Language element of the object to.

Reference numeral 1740 denotes a step for setting a value indicating whether there is a continuation of the message requested to be displayed by the current process in a D_conti element of the object to.

Reference numeral 1750 denotes a step for converting the object to into JSON data and transmitting the converted JSON data to the data conversion computer 230 as a response.

Reference numeral 1760 denotes a step for determining whether there is an acknowledgment from the flavor inhaler or the like within a predetermined time, such as 56 seconds, for example, after the execution of step 1750. In step 1760, it may be determined that there is an acknowledgment when step 1150 is executed after the execution of step 1750. If it is determined that there is an acknowledgment, the process ends, whereas if not, the process returns to step 1750.

### 4-2-8 Refill data retransmission request process

Fig. 18 is a flowchart of at least a part of a refill data retransmission request process 1800.

Reference numeral 1810 denotes a step for determining whether the retransmission of refill data should be requested. The management computer 220 does not have to determine that the retransmission of refill data should be requested if the retransmission of refill data is already requested and the refill data requested to be retransmitted is currently being received. If it is determined that retransmission should be requested, the process proceeds to step 1820, whereas if not, the process ends.

Reference numeral 1820 denotes a step for setting the string "RQ" in the protocol element and the string "RF" in an R Prot element of the object to be used to generate JSON data for requesting the retransmission of refill data.

Reference numeral 1830 denotes a step for setting values indicating the start and stop points of the range of session numbers included in the missing refill data in R_S_Start and R_S_Stop elements, respectively, of the object to.

Reference numeral 1840 denotes a step for converting the object to into JSON data and transmitting the converted JSON data to the data conversion computer 230 as a response.

### 4-2-9 Error data retransmission request process

Fig. 19 is a flowchart of at least a part of an error data retransmission request process 1900.

Reference numeral 1910 denotes a step for determining whether the retransmission of error data should be requested. The management computer 220 does not have to determine that the retransmission of error data should be requested if the retransmission of error data is already requested and the error data requested to be retransmitted is currently being received. If it is determined that retransmission should be requested, the process proceeds to step 1920, whereas if not, the process ends.

Reference numeral 1920 denotes a step for setting the string "RQ" in the protocol element and the string "ER" in the R Prot element of the object to be used to generate JSON data for requesting the retransmission of error data.

Reference numeral 1930 denotes a step for setting values indicating the start and stop points of the range of session numbers included in the missing error data in R_S_Start and R_S_Stop elements, respectively, of the object to.

Reference numeral 1940 denotes a step for converting the object to into JSON data and transmitting the converted JSON data to the data conversion computer 230 as a response.

### 4-2-10 Refill type identification process

Fig. 20 is a flowchart of at least a part of a refill type identification process 2000.

Reference numeral 2010 denotes a step for setting a variable i used in the following steps to 0.

Reference numeral 2020 denotes a step for setting, in a variable refill, information indicating an object for an i-th type of refill from an array refills of which each element indicates each of the objects for a plurality of types of refills stored in advance. In the present embodiment, a single object is an object including an array codes of which each element indicates each of a plurality of color codes for a single type of refill and an element type (which may indicate a brand name of tobacco in the case where the refill is a tobacco capsule, for example), and the data structure refills is an array of which each element indicates the objects for each type of the plurality of types of refills, but it should be noted that this is an example, and information about a plurality of types of refills can be stored using any data structure, such as a table or a list.

Hereinafter, the technical significance of having one or more color codes associated with a single type of refill will be explained. Sensor calibration is performed when the flavor inhaler or the like 210 is shipped from the factory, and even if variations are kept within a certain range, influences such as variations in the refills themselves (for example, color material blending variations in a plastic raw material), measurement error, and ambient light cause variations to occur in the color codes obtained from the sensor output, even for the same type of refill. For this reason, it is preferable if one color code indicating a standard color and one or more color codes indicating approximate colors to the standard color are associated with a single type of refill. Similar colors to the standard color may be determined by any method, such as by experiment. For example, in the case where the color code of the standard color is "da70d6", the approximate color codes may be "ba55d3", "da70d6", "db7093", "dda0dd", "ee82ee", and "fD8080".

Reference numeral 2030 denotes a step for setting a variable j used in the following steps to 0.

Reference numeral 2040 denotes a step for determining whether the color code (the values of the Refill 1 code, Refill_2_code, and Refill_3_code elements of the object ro) being used to attempt to determine the type of refill matches the j-th color code of the object refill. If the color codes match, the process proceeds to step 2045, whereas if not, the process proceeds to step 2050.

Reference numeral 2045 denotes a step for determining the value of the type element of the object indicated by the variable refill as the type of the refill.

Reference numeral 2050 denotes a step for incrementing the value of the variable j by 1.

Reference numeral 2060 denotes a step for determining whether the value of the variable j is less than the size of the array element codes of the data structure indicated by the variable refill. If the value of the variable j is less than the size of the array element codes of the data structure indicated by the variable refill, the process returns to step 2040, whereas if not, the process proceeds to step 2070.

Reference numeral 2070 denotes a step for incrementing the value of the variable i by 1.

Reference numeral 2080 denotes a step for determining whether the value of the variable i is less than the size of the array refills. If the value of the variable i is less than the size of the array refills, the process returns to step 2030, whereas if not, the process proceeds to step 2085.

Reference numeral 2085 denotes a step for executing a predetermined error process. Note that reaching step 2085 means that a refill having a color code that matches the color code being used to attempt to determine the type of refill is not found.

### 4-3 Processes executed in data conversion computer 230

Hereinafter, processes executed in the data conversion computer 230 in one embodiment will be described.

### 4-3-1 Upstream conversion process

Fig. 21 is a flowchart of at least a part of a process (hereinafter referred to as an "upstream conversion process") 2100 for generating JSON data to be transmitted to the management computer 220, the process 2100 being triggered by the reception of binary data from the flavor inhaler or the like 210.

Reference numeral 2110 denotes a step for determining whether the values of bytes 0 and 1 of the binary data from the flavor inhaler or the like 210 are 0x49 and 0x44, respectively (the ASCII codes for the characters "I" and "D", respectively). If the values of bytes 0 and 1 of the binary data are 0x49 and 0x44, respectively, it is determined that the binary data is binary data for initial communication and the process proceeds to step 2115, whereas if not, the process proceeds to step 2120.

Reference numeral 2115 denotes a step for executing a process (hereinafter referred to as an "initial communication conversion process") for converting binary data for initial communication into JSON data for initial communication. The initial communication conversion process will be described later.

Reference numeral 2120 denotes a step for determining whether the values of bytes 0 and 1 of the binary data from the flavor inhaler or the like 210 are 0x52 and 0x46, respectively (the ASCII codes for the characters "R" and "F", respectively). If the values of bytes 0 and 1 of the binary data are 0x52 and 0x46, respectively, it is determined that the binary data is binary data for normal communication and the process proceeds to step 2125, whereas if not, the process proceeds to step 2130.

Reference numeral 2125 denotes a step for executing a process (hereinafter referred to as a "normal communication conversion process") for converting binary data for normal communication into JSON data for normal communication. The normal communication conversion process will be described later.

Reference numeral 2130 denotes a step for determining whether the values of bytes 0 and 1 of the binary data from the flavor inhaler or the like 210 are 0x45 and 0x52, respectively (the ASCII codes for the characters "E" and "R", respectively). If the values of bytes 0 and 1 of the binary data are 0x45 and 0x52, respectively, it is determined that the binary data is binary data for emergency communication and the process proceeds to step 2135, whereas if not, it is determined that the binary data is binary data for acknowledgment communication and the process proceeds to step 2140.

Reference numeral 2135 denotes a step for executing a process (hereinafter referred to as an "emergency communication conversion process") for converting binary data for emergency communication into JSON data for emergency communication. The emergency communication conversion process will be described later.

Reference numeral 2140 denotes a step for executing a process (hereinafter referred to as an "acknowledgment communication conversion process") for converting binary data for acknowledgment communication into JSON data for acknowledgment communication. The acknowledgment communication conversion process will be described later.

### 4-3-2 Initial communication conversion process

Fig. 22 is a flowchart of at least a part of an initial communication conversion process 2200.

Reference numeral 2210 denotes a step for setting the string "ID" in the protocol element of an object o used to generate JSON data for initial communication.

Reference numeral 2220 denotes a step for setting a string expressing a model name generated on the basis of the values of bytes 2 to 4 of the received binary data in a Model element of the object o.

Reference numeral 2230 denotes a step for setting a traceability code generated on the basis of the values of bytes 5 to 9 of the received binary data in a T_code element of the object o. This step can include a step for converting the values of the five bytes into a 12-digit numeral expressed in decimal.

Reference numeral 2240 denotes a step for converting the object o into JSON data and transmitting the converted JSON data to the management computer 220.

### 4-3-3 Normal communication conversion process

Fig. 23 is a flowchart of at least a part of a normal communication conversion process 2300.

Reference numeral 2310 denotes a step for setting the string "RF" in the protocol element of an object o used to generate JSON data for normal communication.

Reference numeral 2320 denotes a step for setting a color code generated on the basis of the values of bytes 2 to 4 of the received binary data in a Refill 1 code element of the object o.

Reference numeral 2330 denotes a step for setting a color code generated on the basis of the values of bytes 5 to 7 of the received binary data in a Refill_2_code element of the object o.

Reference numeral 2340 denotes a step for setting a color code generated on the basis of the values of bytes 8 to 10 of the received binary data in a Refill_3_code element of the object o.

Steps 2320 to 2340 can include a step for converting the value of each of the three bytes into a two-digit numeral expressed in hexadecimal and then concatenating the numerals to obtain a string.

Reference numeral 2350 denotes a step for setting the value of byte 11 of the received binary data as a three-digit numeral in a Session RF element of the object o.

Reference numeral 2360 denotes a step for converting the object o into JSON data and transmitting the converted JSON data to the management computer 220.

### 4-3-4 Emergency communication conversion process

Fig. 24 is a flowchart of at least a part of an emergency communication conversion process 2400.

Reference numeral 2410 denotes a step for setting the string "ER" in the protocol element of an object o used to generate JSON data for normal communication.

Reference numeral 2420 denotes a step for setting an error code generated on the basis of the values of bytes 2 and 3 of the received binary data in an Error code 1 element of the object o.

Reference numeral 2430 denotes a step for setting an error code generated on the basis of the values of bytes 4 and 5 of the received binary data in an Error_code_2 element of the object o.

Reference numeral 2440 denotes a step for setting an error code generated on the basis of the values of bytes 6 and 7 of the received binary data in an Error_code_3 element of the object o.

Reference numeral 2450 denotes a step for setting an error code generated on the basis of the values of bytes 8 and 9 of the received binary data in an Error_code_4 element of the object o.

Steps 2420 to 2450 can include a step for converting the value of each of the two bytes into a character interpreted as an ASCII code and then concatenating the characters to obtain a string.

Reference numeral 2460 denotes a step for setting the value of byte 10 of the received binary data as a three-digit numeral in a Session ER element of the object o.

Reference numeral 2470 denotes a step for converting the object o into JSON data and transmitting the converted JSON data to the management computer 220.

### 4-3-5 Acknowledgment communication conversion process

Fig. 25 is a flowchart of at least a part of an acknowledgment communication conversion process 2500.

Reference numeral 2510 denotes a step for setting the string "AC" in the protocol element of an object o used to generate JSON data for acknowledgment communication.

Reference numeral 2520 denotes a step for converting the value of each of bytes 2 and 3 of the received binary data into a character interpreted as an ASCII code and then setting a string obtained by concatenating the characters in a Response element of the object o. According to this step, for example, the string "DC" is set in the Response element of the object o if the values of bytes 2 and 3 of the received binary data are 0x44 and 0x43, and the string "MS" is set if the values are 0x4D and 0x52.

Reference numeral 2530 denotes a step for determining whether the value of the Response element of the object o is "MS". If the value of the Response element of the object o is "MS", it is determined that the received binary data is binary data for communicating the acknowledgment of a message display request and the process proceeds to step 2535, whereas if not, it is determined that the received binary data is binary data for communicating the acknowledgment of various settings request and the process proceeds to step 2540.

Reference numeral 2535 denotes a step for setting the value of byte 4 of the received binary data in an MS Num element of the object o.

Reference numeral 2540 denotes a step for converting the object o into JSON data and transmitting the converted JSON data to the management computer 220.

### 4-3-6 Downstream communication conversion process

Fig. 26 is a flowchart of at least a part of a process (hereinafter referred to as a "downstream conversion process") 2600 for generating binary data to be transmitted to the flavor inhaler or the like 210, the process 2600 being triggered by the reception of JSON data from the management computer 220.

Reference numeral 2610 denotes a step for converting JSON data jsonDATA from the management computer 220 into an object o.

Reference numeral 2620 denotes a step for determining whether the value of a Protocol element of the object o is the string "UT". If the value of the Protocol element of the object o is the string "UT", it is determined that the received JSON data is JSON data for the request of time setting and the process proceeds to step 2625, whereas if not, the process proceeds to step 2630.

Reference numeral 2625 denotes a step for executing a process (hereinafter referred to as a "time setting request conversion process") for converting JSON data for the request of time setting into binary data for the request of time setting. The time setting request conversion process will be described later.

Reference numeral 2630 denotes a step for determining whether the value of the Protocol element of the object o is the string "DC". If the value of the Protocol element of the object o is the string "DC", it is determined that the received JSON data is JSON data for the request of various settings and the process proceeds to step 2635, whereas if not, the process proceeds to step 2640.

Reference numeral 2635 denotes a step for executing a process (hereinafter referred to as a "various settings request conversion process") for converting JSON data for the request of various settings into binary data for the request of various settings. The various settings request conversion process will be described later.

Reference numeral 2640 denotes a step for determining whether the value of the Protocol element of the object o is the string "MS". If the value of the Protocol element of the object o is the string "MS", it is determined that the received JSON data is JSON data for the request of a message display and the process proceeds to step 2645, whereas if not, it is determined that the received JSON data is JSON data for requesting retransmission and the process proceeds to step 2650.

Reference numeral 2645 denotes a step for executing a process (hereinafter referred to as a "message display request conversion process") for converting JSON data for the request of a message display into binary data for the request of a message display. The message display request conversion process will be described later.

Reference numeral 2650 denotes a step for executing a process (hereinafter referred to as a "retransmission request conversion process") for converting JSON data for requesting retransmission into binary data for requesting retransmission. The retransmission request conversion process will be described later.

### 4-3-7 Time setting request conversion process

Fig. 27 is a flowchart of at least a part of a time setting request conversion process 2700.

Reference numeral 2710 denotes a step for setting the ASCII codes of the characters "U" and "T" in bytes 0 and 1, respectively, of binary data b to be transmitted to the flavor inhaler or the like 210.

Reference numeral 2720 denotes a step for setting a variable i used in the following steps to 0, converting the value of a G_time element of the object o into a numerical value interpreted as a numeral expressed in decimal, and setting the numerical value in a variable time to be used in the following steps.

Reference numeral 2730 denotes a step for extracting the value from the (i*8)th digit to the (i*8+7)th digit when the value of the variable time is expressed in binary and setting numerical value converted from the extracted value in byte i-5 of the binary data b.

Reference numeral 2740 denotes a step for incrementing the value of the variable i by 1.

Reference numeral 2750 denotes a step for determining whether the value of the variable i is less than 4. If the value of the variable i is less than 4, the process returns to step 2730, whereas if not, the process proceeds to step 2760.

Reference numerals 2730 to 2750 denote steps for expressing the value of the variable time in the four bytes of bytes 2 to 5 of the binary data b.

Reference numeral 2760 denotes a step for setting 0 in bytes 6 and 7 of the binary data b. This step is an example of a step for padding the binary data b with appropriate data to make a data size 8 bytes to be transmitted to the flavor inhaler or the like 210.

Reference numeral 2770 denotes a step for transmitting the binary data b to the flavor inhaler or the like 210.

### 4-3-8 Various settings request conversion process

Fig. 28 is a flowchart of at least a part of a various settings request conversion process 2800.

Reference numeral 2810 denotes a step for setting the ASCII codes of the characters "D" and "C" in bytes 0 and 1, respectively, of the binary data b to be transmitted to the flavor inhaler or the like 210.

Reference numeral 2820 denotes a step for setting the values of Parameter 2 to Parameter 0 elements of the object o in bytes 2 to 4, respectively, of the binary data b.

Reference numeral 2830 denotes a step for setting the values (in the present embodiment, 0 or 1) of Flag_7 to Flag_0 elements of the object o in each bit, respectively, of byte 5 of the binary data b.

Reference numeral 2840 denotes a step for setting the value of an F_message element of the object o in byte 6 of the binary data b.

Reference numeral 2850 denotes a step for converting the value of a T diff element of the object o into a numerical value interpreted as a numeral expressed in hexadecimal and setting the numerical value in byte 7 of the binary data b.

Reference numeral 2860 denotes a step for transmitting the binary data b to the flavor inhaler or the like 210.

### 4-3-9 Message display request conversion process

Fig. 29 is a flowchart of at least a part of a message display request conversion process 2900.

Reference numeral 2910 denotes a step for setting the ASCII codes of the characters "M" and "S" in bytes 0 and 1, respectively, of the binary data b to be transmitted to the flavor inhaler or the like 210.

Reference numeral 2920 denotes a step for setting a variable i used in the following steps to 0.

Reference numeral 2930 denotes a step for setting the ASCII code of the i-th character of a C_message element of the object o in byte i+2 of the binary data b.

Reference numeral 2940 denotes a step for incrementing the value of the variable i by 1.

Reference numeral 2950 denotes a step for determining whether the value of the variable i is less than 5. If the value of the variable i is less than 5, the process returns to step 2930, whereas if not, the process proceeds to step 2960.

Reference numerals 2930 to 2950 denote steps for setting the ASCII code of each of the first five characters of the C_message element of the object o in bytes 2 to 6, respectively, of the binary data b.

Reference numeral 2960 denotes a step for setting the values (in the present embodiment, 0 or 1) of a Language element and a D conti element of the object o in bits 1 and 0, respectively, of byte 7 of the binary data b. This step may include a step for setting a predetermined value (for example, 0) in bits 7 to 2 of byte 7 of the binary data b.

Reference numeral 2770 denotes a step for transmitting the binary data b to the flavor inhaler or the like 210.

### 4-3-10 Retransmission request conversion process

Fig. 30 is a flowchart of at least a part of a retransmission request conversion process 3000.

Reference numeral 3010 denotes a step for setting the ASCII codes of the characters "R" and "Q" in bytes 0 and 1, respectively, of the binary data b to be transmitted to the flavor inhaler or the like 210.

Reference numeral 3020 denotes a step for setting a variable i used in the following steps to 0.

Reference numeral 3030 denotes a step for setting the ASCII code of the i-th character of an R Prot element of the object o in byte i+2 of the binary data b.

Reference numeral 3040 denotes a step for incrementing the value of the variable i by 1.

Reference numeral 3050 denotes a step for determining whether the value of the variable i is less than 2. If the value of the variable i is less than 2, the process returns to step 3030, whereas if not, the process proceeds to step 3060.

Reference numerals 3030 to 3050 denote steps for setting the ASCII code of each of the first two characters of the R Prot element of the object o in bytes 2 and 3, respectively, of the binary data b. Note that in the present embodiment, "RF" or "ER" is set in the R Prot element of the object o, with 0x52 and 0x46 being set in the former case and 0x45 and 0x52 being set in the latter case in bytes 2 and 3, respectively, of the binary data b.

Reference numeral 3060 denotes a step for setting the values of R S Start and R_S_Stop elements of the object o as numerical values in bytes 4 and 5, respectively, of the binary data b.

Reference numeral 3070 denotes a step for setting 0 in bytes 6 and 7 of the binary data b. This step is for padding the binary data b with appropriate data to make a data size 8 bytes to be transmitted to the flavor inhaler or the like 210.

Reference numeral 3080 denotes a step for transmitting the binary data b to the flavor inhaler or the like 210.

### 5 Computer

Hereinafter, an example of a hardware configuration of a computer that can be used to carry out one embodiment of the present invention will be described. Note that the computer that can be used to carry out one embodiment of the present invention may be any computer, such as a personal computer or a computer in the cloud, for example. Moreover, the flavor inhalers or the like 100A and 100B (210) described above can be also considered to be computers.

Fig. 31 illustrates an example of a hardware configuration of a computer. As illustrated in the diagram, a computer 3100 is mainly provided with a processor 3110, a main storage 3120, an auxiliary storage 3130, an input-output interface 3140, and a communication interface 3150 as hardware resources, the hardware resources being interconnected through a bus line 3160 including an address bus, a data bus, a control bus, and the like. Note that in some cases, an appropriate interface circuit (not illustrated) may be interposed between the bus line 3160 and each hardware resource.

The processor 3110 is a CPU, microprocessor, or the like that controls all or at least a part of the computer. Note that in some cases, a single computer may include a plurality of processors 3110. In such cases, the "processor" in the above description may be a collective term for the plurality of processors 3110.

The main storage 3120 is a volatile memory such as static random access memory (SRAM) or dynamic random access memory (DRAM) that provides a work area to the processor 3110.

The auxiliary storage 3130 is a non-volatile memory such as an HDD, an SSD, or a flash memory that stores software, that is, a program or the like, and data or the like. The program and data or the like is loaded from the auxiliary storage 3130 into the main storage 3120 through the bus line 3160 at any time. The auxiliary storage 3130 may also be referred to as a computer-readable memory medium, a non-transitory computer-readable memory medium, or a computer-readable storage medium. Note that the program includes instructions causing the processor to execute desired processes.

The input-output interface 3140 presents information and/or receives the input of information, and is a digital camera, a keyboard, a mouse, a display, a touch panel display, a microphone, a speaker, any of various types of sensors, and the like.

The communication interface 3150 is connected to a network 3155, and transmits and receives data over the network 3155. The communication interface 3150 and the network 3155 may be connected in a wired or wireless way. The communication interface 3150 may also obtain network-related information, such as information pertaining to a Wi-Fi access point and information related to a base station of a communications carrier, for example.

It should be obvious to a person skilled in the art that, through the cooperation of the hardware resources and software exemplified above, the computer 3100 functions as desired means, executes desired steps, and causes desired functions to be achieved.

### 6 Conclusion

The above describes several examples of embodiments of the present invention, but it should be understood that the above examples are merely for illustrative purposes and do not limit the technical scope of the present invention. It should be understood that changes, additions, improvements, and the like can be made to the embodiments without departing from the gist and scope of the present disclosure. The technical scope of the present invention should not be limited by any of the embodiments described above, but should be defined only by the claims and their equivalents.

### REFERENCE SIGNS LIST

100A, 100B flavor inhaler or the like
121A heating component
122 liquid channeling component
124 mouthpiece
140 holding component
141 internal space
142 opening
143 base component
144 insulating component
150 stick-type substrate
151 substrate component
152 inhaling component
180 air channel
181 air inflow hole
182 air outflow hole
190 arrow
200 system
240 LPWA network
400 initial communication process
500 normal communication process
600 emergency communication process
700 response analysis process
800 retransmission process
900 various settings process
1000 message display process
1100 response process
1200 initial communication response process
1300 normal communication or the like response process
1400 emergency communication or the like response process
1500 acknowledgment communication response process
1600 various settings request process
1700 message display request process
1800 refill data retransmission request process
1900 error data retransmission request process
2000 refill type identification process
2100 upstream conversion process
2200 initial communication conversion process
2300 normal communication conversion process
2400 emergency communication conversion process
2500 acknowledgment communication conversion process
2600 downstream communication conversion process
2700 time setting request conversion process
2800 various settings request conversion process
2900 message display request conversion process
3000 retransmission request conversion process
3155 network
3160 bus line

## Claims

1. A device which is a flavor inhaler or an aerosol-generating device, the device being configured to:
defer a process for transmitting data, if control data for controlling the device is received when the process is on standby;
transmit acknowledgment data; and
execute the process for transmitting data.

2. The device according to claim 1, wherein the device is further configured to:
obtain data which is at a predetermined position in the control data and which has a predetermined size;
perform a first control on a basis of first data from the control data, wherein the size of the first data is one or more bytes, the first data is in units of bytes, and the size and a position of the first data in the control data is determined on a basis of the obtained data; and
perform a second control on a basis of second data, which is one bit, from the control data, wherein a position of the second data in the control data is determined on a basis of the obtained data.

3. The device according to claim 2, wherein
the first data includes a message encoded in extended ASCII code, and
the first control includes a message display.

4. The device according to any one of claims 1 to 3, wherein the device is further configured to:
perform communication in accordance with a predetermined LPWA wireless communication standard at least to receive the control data and transmit the acknowledgment data.

5. A management computer configured to:
transmit control data for controlling a device which is a flavor inhaler or an aerosol-generating device, wherein
the transmitted control data is retransmitted if acknowledgment data is not received within a predetermined time from the transmission of the control data.

6. The management computer according to claim 5, further configured to:
transmit control data including a portion of an encoded message; and
if the acknowledgment data is received within a predetermined time from the transmission of the control data, transmit control data including a different portion of the encoded message.

7. A data conversion computer for generating control data in a first format on a basis of control data in a second format to control a device which is a flavor inhaler or an aerosol-generating device, the data conversion computer being configured to:
generate, on a basis of data being a first portion of the control data in the second format, first data the size of which is one or more bytes and which is in units of bytes;
generate, on a basis of data being a second portion of the control data in the second format, second data, the size of which is one bit;
generate, on a basis of one or more sets of second data, third data, the size of which is one byte; and
generate, on a basis of the first data and the third data, the control data in the first format.

8. The data conversion computer according to claim 7, further configured to:
obtain data being a predetermined third portion of the control data in the second format, wherein
which portions of the control data in the second format are the first portion and the second portion are determined on a basis of the obtained data being the third portion,
the generating of the third data, the size of which is one byte, includes:
arranging the one or more sets of second data in an order determined on a basis of the obtained data being the third portion, and
the control data in the first format is generated also on a basis of the obtained data being the third portion.

9. A system comprising:
the device according to any one of claims 1 to 5;
the management computer according to claim 5 or 6; and
the data conversion computer according to claim 7 or 8, wherein
control data received by the device is the control data in the first format generated by the data conversion computer, and
control data in the second format used by the data conversion computer is the control data transmitted by the management computer.

10. A method executed by a device which is a flavor inhaler or an aerosol-generating device, the method comprising:
deferring a process for transmitting data, if control data for controlling the device is received when the process is on standby;
transmitting acknowledgment data; and
executing the process for transmitting data.

11. A method executed by a computer, the method comprising:
transmitting control data for controlling a device which is a flavor inhaler or an aerosol-generating device; and
retransmitting the transmitted control data if acknowledgment data is not received within a predetermined time from the transmission of the control data.

12. A method executed by a computer for generating control data in a first format on a basis of control data in a second format to control a device which is a flavor inhaler or an aerosol-generating device, the method comprising:
generating, on a basis of data being a first portion of the control data in the second format, first data, the size of which is one or more bytes and which is in units of bytes;
generating, on a basis of data being a second portion of the control data in the second format, second data, the size of which is one bit;
generating, on a basis of one or more sets of the second data, third data, the size of which is one byte; and
generating, on a basis of the first data and the third data, the control data in the first format.

13. A program causing a device which is a flavor inhaler or an aerosol-generating device to execute the method according to claim 10.

14. A program causing a computer to execute the method according to claim 11 or 12.
